# EUROPEAN PATENT SPECIFICATION

(11) **EP 1 194 777 B1**
(45) Date of publication and mention of the grant of the patent: **22.11.2006**
(21) Application number: 00935381.4
(22) Date of filing: 01.06.2000
(51) Int. Cl.: G01N 33/50, G01N 33/68, C07K 14/47

(54) **METHODS AND COMPOSITIONS RELATING TO PANCREATIC ISLET AND BETA-CELL DYSFUNCTION**
METHODEN UND ZUSAMMENSETZUNGEN BEZÜGLICH PANKREATISCHER INSELZELLEN- UND BETA-ZELLEN- DYSFUNKTION
PROCEDES ET COMPOSITIONS SE RAPPORTANT AU DYSFONCTIONS DES ILOTS PANCREATIQUES ET DES CELLULES BETA

(30) Priority: 25.06.1999 GB 9914890
(43) Date of publication of application: 10.04.2002
(73) Proprietor: PROTEOME SCIENCES PLC, Cobham, Surrey KT11 3EP (GB); University of Geneva, 1211 Geneva 4 (CH)
(72) Inventor: CAWTHORNE, Michael, Hunter Street Buckingham MK18 1EG (GB); SANCHEZ, Jean-Charles, CH-1208 Geneva (CH)
(74) Representative: Kiddle, Simon John
(86) International application number: PCT/GB2000/002118
(87) International publication number: WO 2001/001130

(56) References cited:
- WO-A-00/46369
- WO-A-97/15310
- EDVARDSSON ULRIKA ET AL: "A proteome analysis of livers from obese (ob/ob) mice treated with the peroxisome proliferator WY14,643." ELECTROPHORESIS, vol. 20, no. 4-5, April 1999 (1999-04), pages 935-942, XP000964564 ISSN: 0173-0835
- SHIMADA A ET AL: "BETA-CELL DESTRUCTION MAY BE A LATE CONSEQUENCE OF THE AUTOIMMUNE PROCESS IN NONOBESE DIABETIC MICE" DIABETES,US,NEW YORK, NY, vol. 45, no. 8, 1 August 1996 (1996-08-01), pages 1063-1067, XP000603754 ISSN: 0012-1797

## Description

### Field of the Invention

The present invention relates to methods and compositions relating to pancreatic islet and β-cell dysfunction, in conditions such as non-insulin dependent diabetes. Specifically, the present invention identifies and describes proteins that are differentially expressed in non-insulin dependent diabetes relative to their expression in the normal state and, in particular, identifies and describes proteins associated with pancreatic islet or β-cell dysfunction and/or mass. Further, the present invention identifies and describes proteins via their ability to interact with gene products involved in the regulation of pancreatic islets or β-cell dysfunction and/or mass. Still further, the present invention provides methods, particularly experimental paradigms, for the identification of differential expressed proteins that are potential molecular targets for compounds to treat or prevent pancreatic islets or β-cell dysfunction and non-insulin dependent diabetes mellitus. Still further, the present invention provides methods for the identification and therapeutic use of compounds for the prevention and treatment of pancreatic islet or β-cell dysfunction and non-insulin dependent diabetes mellitus.

### Background of the Invention

Diabetes mellitus is one of the most common metabolic disorders affecting more than 100 million people worldwide (King, H. and Zimmet, P. (1988) World Health Statistics Quarterly 41, 190-196; Harris, M.I. et al (1992) Diabetes Care 15, 815-819). It is predicted that the world incidence of diabetes will double by year 2010 largely through an increase in incidence in industrially developing countries such as India, China, South America and the Far East.

There are two types of diabetes. Type I, or insulin dependent diabetes mellitus (IDDM), is the result of progressive autoimmune destruction of the pancreatic β-cells and constitutes 5-10% of the total diabetic population. Type II diabetes, or non-insulin dependent diabetes mellitus (NIDDM) or maturity onset diabetes, represents 90-95% of the diabetic population. Typically it occurs in middle-aged and elderly subjects, although it can develop in younger subjects and it is commonly associated with obesity. NIDDM is associated with two metabolic defects: insulin resistance and inappropriate insulin secretion. The insulin resistance affects muscle and adipose tissues, resulting in reduced glucose uptake and elevated hepatic production of glucose. The pancreatic lesion is an essential component of NIDDM. Typically the initial lesion appears to be an overproduction of insulin in response to a glucose load, but later there is a loss or reduction in the first phase insulin secretion response and eventually the insulin secretion capacity declines to such an extent that exogenous insulin needs to be administered. Family studies indicate a major genetic component in the development of NIDDM, but apart from a sub-type of NIDDM called maturity onset diabetes of the young (MODY), few susceptibility genes have been identified.

Traditionally treatment for non-insulin dependent diabetes mellitus has focussed on the control of blood glucose. Current drugs, however, generally fail to achieve the same degree of control of blood glucose as is present in a non-diabetic subject. In addition, NIDDM patients often have elevated plasma triglycerides and elevated plasma cholesterol or a low ratio of HDL:LDL-cholesterol. All of these metabolic changes are adverse with respect to the development of the secondary complications of diabetes, which includes cardiovascular disease, blindness, nephropathy, stroke and microvascular disease.

There remains a pressing need in the art for more effective treatments for non-insulin dependent diabetes.

The diabetic population is extremely heterogenous and it is likely that the development of β-cell dysfunction and NIDDM has multiple causes. In part, it probably relates to a failure to meet the increased insulin requirement and arises as a result of insulin resistance. However, other factors including toxins, viral disease and genetically inherited defects cannot be ruled out.

There are a number of animal models with mutations that are associated with insulin resistance and attempts have been made to utilise such animals as models for the study of non-insulin dependent diabetes, insulin resistance and pancreatic β-cell function. The best studied animal models for insulin resistance and non-insulin dependent diabetes are mice, which contain the autosomal recessive mutations ob/ob (obese) and db/db (diabetes). These mutations are on chromosome 6 and 4 respectively, but lead to clinically similar pictures provided the genes are expressed on the same background strain. The ob gene product has been identified as 16kDa polypeptide produced primarily by adipose tissue that provides a signal to the brain on the adipose tissue fat stores. Mice with a mutation, resulting in no circulating protein (called leptin) are hyperphagic, obese, have poor thermoregulation and non-shivering thermogenesis and are insulin resistant with impaired glucose tolerance. When the OB gene mutation is on the C5BI/6 background the mice do not present with frank clinically evident diabetes, but are massively hyperinsulinaemic and glucose intolerant.

The db/db mice have a mutation in the receptor for leptin so that normal signal transduction via the JAK/STAT pathway does not occur. This mutation, when on the C57BI/6 background, is phenotypically identical to the ob mutation. However, the db/db mutation is normally expressed on the C57BI/Ks mouse background and on this background the mutation causes frank diabetes. It is recognised that a major difference between wild-type mice of the C57BI/6 strain and wild-type mice of the C57BI/Ks strain is that the Bl/6 mice have approximately twice the pancreatic islet cell mass of the Bl/Ks mice. Thus, mice of the C57BI/6 strain are able to withstand the pancreatic β-cell stress imposed by insulin resistance more than mice of the C57B1/Ks strain.

Other mutant animal models include fa/fa (fatty) rats and ZDF fatty rats, which bear strong respective similarities with the ob/ob and db/db mice. Thus the fa/fa rat is obese, insulin resistant, very hyperinsulinaemic and glucose intolerant, whereas the ZDF rat is obese, insulin resistant and hyperinsulinaemic, but the male rats develop frank diabetes after approximately 6 weeks of age. It follows that the pancreatic islets of the Zucker fa/fa rats and ZDF male rats must differ in their ability to maintain insulin secretion in the face of insulin resistance. Similarly, pancreatic islets from ZDF male rats must be intrinsically different from pancreatic islets from ZDF female rats, which do not develop frank diabetes.

Inbred mouse strains, such as the NZO mouse, the Japanese KK mouse and the GK rat are models of insulin resistance, pancreatic β-cell dysfunction and diabetes. Further, desert rodents, such as spiny mice and sand rats are neither insulin resistant nor diabetic in their natural habitats, but do present frank diabetes when fed on a standard laboratory diet.

Insulin resistance, developing pancreatic β-cell dysfunction and glucose intolerance are a common feature of elderly rodents and the development of the defects can be accelerated by feeding diets with a high fat content, whether these diets are synthetic homogenous diets or are the result of supplementation of replacement of the normal rat chow by human food with a high fat content (cafeteria diet).

Human non-insulin dependent diabetes has a high concordance between twins with a higher rate in identical twins than in non-identical twins. There is also a strong familial tendency associated with both parents. Thus, offspring where both parents have NIDDM have a higher risk of developing NIDDM than offspring in which only one parent has NIDDM.

Recent data has shown that there is also a significant correlation between low birth-weight (in relation to gestational age) and the development of non-insulin dependent diabetes in later life. This suggests a possible linkage between in utero nutrition and the development of non-insulin dependent diabetes. The molecular nature of this linkage in human NIDDM has not been defined. However, studies in rats have shown that if pregnant female rats are fed on a 6% protein diet rather than the more usual 15% protein diet, the two sets of pregnant rats produce a similar number of offspring but the pups from the mothers fed on a 6% protein diet are smaller. If these pups are subsequently given post-weaning a high-fat diet to induce insulin resistance, then they develop non-insulin dependent diabetes. Furthermore, it has been demonstrated that the pups from the mothers fed on a 6% protein diet have a smaller islet cell mass than pups from mothers fed on a 15% protein diet.

Much of the current increase in incidence in NIDDM is occurring in developing countries. In such countries it is likely that the incidence of small for gestational age babies will be high. As these countries change from their traditional high carbohydrate, locally grown diet to a more western style high fat diet, as occurs during acculturalisation, the development of insulin resistance in subjects puts increased pressure on pancreatic islet function. The identification of those factors that are associated with the development of a low islet cell mass would allow the discovery of novel agents to prevent the development of non-insulin dependent diabetes as well as treat the pre-existing condition.

Absolute pancreatic β-cell mass is largely determined at birth or shortly after birth. However, the pancreatic β-cell mass is increased in certain situations, for example during pregnancy. Thus a model for examining the molecular nature of proliferative activity of the pancreatic β-cell is the pregnant animal.

The above animal models have been used from time to time to evaluate new drugs that were potential treatments for pancreatic β-cell dysfunction or non-insulin dependent diabetes. However, although individual changes in enzyme activities have been identified in some of the animal models and how this might be altered by a drug therapy, no systematic evaluation has been made of the differences in protein expression in the pancreatic islet or β-cells of normal animals showing β-cell dysfunction. It is these changes in protein expression that underlie the development of β-cell dysfunction leading to non-insulin dependent diabetes. It is the same changes in protein expression that are likely to be causative of non-insulin dependent diabetes in humans and in companion animals such as dogs and cats. Given the severity and prevalence of non-insulin dependent diabetes and pancreatic β-cell dysfunction, there exists a great need for the systematic identification of the disease causing proteins, since modulation of the expression level of such proteins back to the level in non-diabetic individuals represents a means of treating the disease condition.

US Patent 5,702 902 (Tartaglia) identifies and describes genes which are differentially expressed at the mRNA level in body weight disorder states, relative to their expression in normal states. It is suggested therein that gene expression pattern can be used to identify compounds that can be used therapeutically to treat body weight disorders by either altering gene expression or by interacting with the gene products (proteins) of the differentially expressed genes.

The use of differential gene expression as a tool for identifying the molecular basis of a disease process such as insulin resistance disorders relies on the differential gene mRNA expression being directly translated into a differential protein expression. This is not the case. The changes in protein expression is much more complex since the amount of protein present is influenced by the turnover rate of the corresponding mRNA, the turnover rate of the individual proteins, interaction of proteins with binding proteins and post-translational modification such as phosphorylation. Thus it is the changes in protein expression (including post-translational modification) that underlie the development of insulin resistance disorders including non-insulin dependent diabetes. It is these same changes in protein expression that are likely to be causative of insulin resistance disorders including non-insulin dependent diabetes in humans and companion animals such as cats and dogs.

It has been a problem to find a more predictive method for the identification of the molecular basis of insulin resistance and thereby define the molecular targets that can be used to identify agents to treat the disease. It is also a problem to identify from the therapeutic tools available the most appropriate therapy for any individual with insulin resistance disorders given the severity and prevalence of insulin resistance disorders, particularly non-insulin dependent diabetes, there exists a great need for the systematic identification of the disease causing proteins, since modulation of the expression level of such proteins or the activity of such proteins in the subjects with insulin resistance disorders towards the level in normal or non-insulin resistant subjects represents a means of treating the condition. Furthermore, since there are multiple causes of the overall insulin resistant state such methodologies will allow a prognosis to be made of the most appropriate and potentially most effective therapy to treat any individual suffering from the insulin resistant disorder.

### Summary of the Invention

Edvardsson et al. (Electrophoresis 1999, 20, 935-942) have examined the effect of the compound WY14,643 (an agonist of the peroxisome proliferator activated receptor PPARα) on liver protein expression in ob/ob mice by 2-dimensional gel electrophoresis and found upregulation of at least 16 spots, 14 of which were found to be involved in peroxisomal fatty acid metabolism.

The invention provides a method of screening a test agent to determine its usefulness in treating a condition characterised by pancreatic islet or β-cell dysfunction, the method comprising:
(a) establishing a paradigm in which at least one protein is differentially expressed in relevant tissue from, or representative of, subjects having differential levels of pancreatic islet or β-cell function, the subjects comprising:
   (i) normal subjects who have and have not been treated with an agent which improves pancreatic islet or β-cell function, and
   (ii) affected subjects having reduced or increased pancreatic islet or β-cell function relative to the normal subjects, wherein said affected subjects have and have not been treated with the agent which improves pancreatic islet or β-cell function,
   wherein said differentially expressed protein is differentially expressed between said affected and normal subjects who have not been treated with the agent which improves pancreatic islet or β-cell function, and is differentially expressed between said affected subjects who have and have not been treated with the agent which improves pancreatic islet or β-cell function, but is not differentially expressed between said normal subjects who have and have not been treated with the agent which improves pancreatic islet or β-cell function;
(b) providing a sample of relevant tissue which has been obtained from, or is representative of, a test subject having reduced pancreatic islet or β-cell function, who or which has been treated with the test agent being screened;
(c) determining the presence, absence or degree of expression of the differentially expressed protein or proteins in the tissue from, or representative of, the treated test subject; and,
(d) selecting or rejecting the agent according to the extent to which it changes the expression, activity or amount of the differentially expressed protein or proteins in the treated test subject having reduced pancreatic islet or β-cell function.

The paradigm may involve establishing at least one protein which is differentially expressed. However, in some embodiments, the paradigm may employ at least 2, 3, 4, 5, 6, 7, 8, 9, 10 or 20 differentially expressed proteins.

Typically, a test agent is selected if it changes the expression of a differentially expressed protein towards that of a subject having more normal pancreatic islet or β-cell function.

The invention further provides a method of determining the nature or degree of pancreatic islet or β-cell dysfunction in a human or animal test subject, the method comprising:
(a) establishing a paradigm in which at least one protein is differentially expressed in relevant tissue from, or representative of, subjects having differential levels of pancreatic islet or β-cell function, the subjects comprising
   (i) normal subjects who have and have not been treated with an agent which improves pancreatic islet or β-cell function, and
   (ii) affected subjects having reduced or increased pancreatic islet or β-cell function relative to the normal subjects, wherein said affected subjects have and have not been treated with an agent which improves pancreatic islet or β-cell function,
   wherein said differentially expressed protein is differentially expressed between said affected and normal subjects who have not been treated with the agent which improves pancreatic islet or β-cell function, and is differentially expressed between said affected subjects who have and have not been treated with the agent which improves pancreatic islet or β-cell function, but is not differentially expressed between said normal subjects who have and have not been treated with the agent which improves pancreatic islet or β-cell function;
(b) providing a sample of the tissue which has been obtained from the test subject;
(c) determining the presence, absence or degree of expression of the differentially expressed protein or proteins in the sample; and
(d) relating the determination to the nature or degree of the pancreatic islet or β-cell function by reference to a previous correlation between such a determination and clinical information.

Conveniently, the patient sample used in the method can be a tissue sample or body fluid sample or urine. This method allows the type of pancreatic islet or β-cell dysfunction of a patient to be correlated to different types to prophylactic or therapeutic treatment available in the art, thereby enhancing the likely response of the patient to the therapy.

The method may be used to predict the most appropriate and effective therapy to alleviate the pancreatic islet or β-cell dysfunction state and to monitor the success of that treatment.

Examples of differentially expressed protein described herein and found in samples from pancreatic islet or β-cells include Tropomyosin isoform 1 (SWISS-PROT AC P46901), Fatty acid binding protein (adipocyte) (SWISS-PROT AC P04117), Profilin (SWISS-PROT AC P10924) and Carboxypeptidase B (SWISS-PROT AC P19223).

This invention is based, in part, on systematic search strategies involving modulations of pancreatic islet and/or β-cell structure and function and non-insulin dependent diabetes experimental paradigms, coupled with sensitive detection of proteins by 2D-electrophoresis. To aid the identification of differentially expressed protein a standard marker set of proteins such as those available from Genomic Solutions may be added to the islet proteins prior to 2D electrophoresis.

The examples presented below demonstrate the identification of differentially expressed proteins associated with a reduced pancratic islet and/or β-cell mass and proteins associated with pancreatic islet and/or β-cell dysfunction.

### Definitions

"Differential expression", as used herein, refers to at least one recognisable difference in tissue protein expression. It may be a quantitatively measurable, semi-quantitatively estimatable or qualitatively detectable difference in tissue protein expression. Thus, a differentially expressed protein (herein DEP) may be strongly expressed in tissue in the normal state and less strongly expressed or not expressed at all in tissue in the pancreatic islet or β-cell dysfunctional state. Conversely, it may be strongly expressed in tissue in the disorder state and less strongly expressed-or-not expressed at all in the normal state. Similarly, the differential expression can be either way around in the comparison between untreated and treated tissue.
Further, expression may be regarded as differential if the protein undergoes any recognisable change between the two states under comparison.

The term "paradigm" means a prototype example, test model or standard.

Wherever a differentially expressible protein is used in the screening procedure, it follows that there must have been at some time in the past a preliminary step of establishing a paradigm by which the differential expressibility of the protein was pre-determined. Once the paradigm has been established, it need not be reestablished on every occasion that a screening procedure is carried out. The term "establishing a paradigm" is to be construed accordingly.

"Islet or β-cell dysfunction" includes conditions in which the mass of these cell types is reduced in patients and/or where the cells have reduced function as compared to normal cells, e.g. in the production of insulin. Conditions characterised by islet or β-cell dysfunction include non-insulin dependent diabetes or type 2 diabetes, syndrome X or insulin resistance syndrome or gestational diabetes.

"Relevant tissue" means any tissue which undergoes a biological change in response to the action of insulin in the body, or any other tissue affected by this change.

"Tissue... ...representative of... ...subjects" means any tissue in which the above-mentioned biological change can be simulated for laboratory purposes and includes, for example, a primary cell culture or cell line derived ultimately from relevant tissue.

The term "subjects" includes human and animal subjects.

The treatments referred to above can comprise the administration of one or more drugs or foodstuffs, and/or other factors such as diet or exercise.

The term "diagnosis", as used herein, includes the provision of any information concerning the existence, non-existence or probability of the disorder in a patient. It further includes the provision of information concerning the type or classification of the disorder or of symptoms which are or may be experienced in connection with it. It encompasses prognosis of the medical course of the disorder.

The term "alleviate", as used herein, in relation to pancreatic islet or β-cell dysfunction means any form of reducing one or more undesired symptoms or effects thereof. Any amelioration of the pancreatic islet or β-cell dysfunction of the patient falls within the term "alleviation".

By way of example, embodiments of the present invention will now be described in more detail with reference to the accompanying figures.

### Brief Description of the Figures

Figure 1 shows a computer images of stained 2-DGE gels from pancreatic islet cells of lean control mice, identifying spots thereon, including DEPs.
Figures 2 to 7 and Figures 9 to 13 show the comparative expression of the DEPs together with quantification of the differential expression.
Figures 8 and 14 show a control protein (C1230) that is not differentially expressed.

### Detailed Description

Proteins are described which are differentially expressed in pancreatic islet and/or β-cell disorders states relative to their expression in normal states and/or which are differentially expressed in response to manipulations relevant to the regulation of pancreatic islet and/or β-cell mass and/or function.

Described below are methods for the identification of compounds, which modulate the expression of proteins, involved in pancreatic islet and/or β-cell mass and/or function regulation.

Also discussed below are methods for prognostic and diagnostic evaluation of pancreatic islet and/or β-cell disorder states and for the identification of subjects exhibiting a predisposition to such disorders.

### Identification of differentially expressed proteins

'Differential expression', as used herein, refers to both qualitative as well as quantitative differences in protein expression. Thus a differentially expressed protein may qualitatively have its expression activated or completely inactivated in normal versus pancreatic islet and/or β-cell disorder state or under control versus experimental conditions. Such a qualitatively regulated protein will exhibit an expression pattern within a given tissue or cell type, which is detectable in either control or pancreatic islet and/or β-cell disorder subject, but not detectable in both. Alternatively, such a qualitatively regulated protein will exhibit an expression pattern within one or more cell types in the pancreatic islet, which is detectable in either control or experimental subjects but not detectable in both. 'Detectable', as used herein, refers to a protein expression pattern, which are detectable using techniques such as differential display 2D electrophoresis.

Alternatively, a differentially expressed protein may have its expression modulated, i.e. quantitatively increased or decreased, in normal versus pancreatic islet and/or β-cell disorder states or under control versus experimental conditions. The degree to which expression differs in normal versus pancreatic islet and/or β-cell disorder states or control versus experimental states need only be large enough to be visualised via standard characterisation techniques, such as silver staining of 2D-electrophoretic gels. Other such standard characterisation techniques by which expression differences may be visualised are well known to those skilled in the art. These include successive chromatographic separations of fractions and comparisons of the peaks, capillary electrophoresis and separations using micro-channel networks, including on a micro-chip.

Chromatographic separations can be carried out by high performance liquid chromatography as described in Pharmacia literature, the chromatogram being obtained in the form of a plot of absorbance of light at 280 nm against time of separation. The material giving incompletely resolved peaks is then re-chromatographed and so on.

Capillary electrophoresis is a technique described in many publications, for example in the literature "Total CE Solutions" supplied by Beckman with their P/ACE 5000 system. The technique depends on a applying an electric potential across the sample contained in a small capillary tube. The tube has a charged surface, such as negatively charged silicate glass. Oppositely charged ions (in this instance, positive ions) are attracted to the surface and then migrate to the appropriate electrode of the same polarity as the surface (in this instance, the cathode). In this electroosmotic flow (EOF) of the sample, the positive ions move fastest, followed by uncharged material and negatively charged ions. Thus, proteins are separated essentially according to charge on them.

Micro-channel networks function somewhat like capillaries and can be formed by photoablation of a polymeric material. In this technique, a UV laser is used to generate high energy light pulses that are fired in bursts onto polymers having suitable UV absorption characteristics, for example polyethylene terephthalate or polycarbonate. The incident photons break chemical bonds with a confined space, leading to a rise in internal pressure, mini-explosions and ejection of the ablated material, leaving behind voids which form microchannels. The micro-channel material achieves a separation based on EOF, as for capillary electrophoresis. It is adaptable to micro-chip form, each chip having its own sample injector, separation column and electrochemical detector: see J.S. Rossier et al., 1999, Electrophoresis 20: pages 727-731.

### Method for the identification of differentially expressed proteins

A variety of methods may be used for the identification of proteins, which are involved in pancreatic islet and/or β-cell disorder states and/or which may be involved in non-insulin dependent diabetes. Described below are several experimental paradigms, which may be utilised for the generation of subjects, and samples, which may be used for the identification of such proteins. Material from the paradigm control and experimental subjects may be characterised for the presence of differentially expressed protein sequences.

### Paradigms for the identification of differentially expressed proteins

Among the paradigms that may be utilised for the identification of differentially expressed proteins involved in pancreatic islet and/or β-cell disorder states are paradigms designed to analyse those proteins that are differentially expressed between normal and pancreatic islet and/or β-cell disorder states including, but not limited to, non-insulin dependent diabetes, gestational diabetes and impaired glucose tolerance.

In one such paradigm, pancreatic islet tissue from normal and pancreatic islet and/or β-cell disorder subjects would be compared. Such subjects could include, but would not be limited to, subjects with non-insulin dependent diabetes, impaired glucose tolerance, gestational diabetes, first degree relatives of non-insulin dependent diabetes. It could also involve a comparison of normal and pregnant individuals or normal subjects and subjects who have resisted the development of pancreatic islet and/or β-cell disorders despite the presence of insulin resistance. Appropriate tissues would include, but not be limited to, blood and pancreatic islets.

Among additional paradigms would include a comparison of non-insulin diabetic subjects and subjects whose pancreatic islet and/or β-cell function had been improved by, but not limited to, dietary restriction or modification, insulin sensitiser drugs, such as troglitazone and rosiglitazone, metformin, exercise and β₃-adrenoceptor agonists such as BRL 35135 and 26830.

In a further paradigm, which may be utilised for the identification of differentially expressed proteins involved in pancreatic islet and/or β-cell disorder states are paradigms designed to analyse those proteins which may be involved in genetic models of pancreatic islet and/or dysfunction or alterations in β-cell mass and/or non-insulin dependent diabetes. Accordingly, such paradigms are referred to as 'genetic pancreatic islet and/or β-cell paradigms'. In the case of mice, for example, such paradigms may identify the proteins regulated either directly or indirectly by the ob/ob, db/db, tub or fat gene products. In rats, such a paradigm may identify proteins regulated either directly or indirectly by the fa gene product. Such paradigms may also identify the proteins regulated by the genetic background and which result in the diabetic condition when the ob/ob, db/db and fa gene products are expressed on the diabetes sensitive background

The essential difference between the first, second and third types of paradigm is that in the first the paradigm is not established by any treatment regime or drug, since normal subjects (e.g. lean control mice) are being compared with disorder subjects (e.g. obese mice), whereas in the second this comparison is supplemented by a further comparison between disorder subjects, untreated and treated (e.g. obese control mice and obese mice which have been treated with a drug). Thus, for example, when establishing paradigms, using lean and obese mice, with rosiglitazone as the drug for treatment, it is convenient to run four experimental groups at once, with lean control mice, obese control mice, lean treated mice and obese treated mice. The DEPs can then be grouped as follows:
Group 1 ("OM"): DEPs in the lean control v. obese control comparison, but which are NOT Group 2 DEPs.
Group 2 ("OMT"): DEPs in the lean control v. obese control comparison which are also DEPs in the obese treated v. obese control comparison, but which are NOT DEPs in a lean control v. lean treated comparison.
Group 3 ("SEM"): DEPs in the obese treated v. obese control comparison, but which are also DEPs in the lean treated v. lean control comparison.

It will be appreciated that each such type 1, type 2 or type 3 paradigm as illustrated above relates to a particular treatment, in this case with rosiglitazone. When another drug "X" (known or yet to be discovered) is used to establish a paradigm, it can be expected that there will be some differences. For example, some DEPs which are in Group 1, being "rosiglitazone-insensitive", may additionally be sensitive to drug "X" and therefore appear in Group 2 in the new paradigm. If, further, the lean treated v. lean control comparison of this DEP shows no major difference, it will be placed in Group 2.

In one embodiment of such a paradigm, test subjects may include ob/ob, db/db, tub/tub or fat/fat experimental mice and lean littermate controls on both the C57BI/6 and C57BI/Ks backgrounds. Test subjects could also include fa/fa and male and female ZDF rats. Samples of pancreas would be obtained and islets prepared free of exocrine pancreas. The examples provided below demonstrate the use of such genetic paradigms in identifying proteins which are differentially expressed in pancreatic islet and/or β-cell disorder animals versus normal animals.

In additional embodiments, ob/ob, db/db, tub/tub and/or fat/fat mice and/or fa/fa and ZDF rats and lean control animals may be treated with drugs that improve pancreatic islet and/or β-cell function. Such drugs include, but are not limited to, insulin sensitisers such as the thiazolidinediones, rosiglitazone, pioglitazone or troglitazone, the oxazolidadiones JTT501, non-thiazolidinedione PPAR gamma activators, RXR activators that form heterodimers with PPAR gamma, metformin and b₃₋adrenoceptor agonists. Such a paradigm allows the identification of target proteins.

In a further additional embodiment, ob/ob, db/db, tub/tub and/or fat/fat mice and/or fa/fa and/or ZDF rats and lean controls may be offered dietary treatments to either worsen the insulin resistant state or improve insulin sensitivity. For example, either lean or insulin resistant animals could be provided with a high fat diet to exacerbate the insulin resistant state.

In one embodiment of such a paradigm, C57BI/6 and C57BI/Ks wild-type mice would be fed on a high fat diet or a cafeteria diet consisting of human snack foods. The C56BI/Ks mice have a smaller pancreatic islet cell mass than the C57BI/6 mice. Hence when demand for insulin secretion is increased by the development of insulin resistance by feeding on a high fat or cafeteria diet, the C57BI/Ks mice are compromised more. Thus, this paradigm can be used to select proteins that are associated with a predisposition to a pancreatic islet and/or β-cell disorder state. The paradigm can be further refined by incorporating drug treatment paradigms.

Some native animal strains do not exhibit either pancreatic islet and/or β-cell dysfunction or non-insulin dependent diabetes in the wild but do when fed a laboratory chow or other laboratory diets. These include the desert rodents, the spiny mouse and the sand rat.

Comparison of animals fed on the natural diet and those fed on a laboratory diet allows identification of proteins associated with pancreatic islet and/or β-cell disorders.

Further paradigms that may be utilised for the identification of differentially expressed protein involved in pancreatic islet and/or β-cell disorder states may include paradigms in which pregnant mice or rats may be fed on a low protein diet (typically 6% fat) during pregnancy and/or lactation whereas control animals are fed on a diet containing a normal protein content (typically 15%). After weaning, offspring may be fed on a high fat diet. Such latter offspring develop non-insulin dependent diabetes. Comparison between these various animals fed on different diets during gestation, weaning and/or in adult life allows the identification of differentially expressed proteins associated with pancreatic islet and/or β-cell dysfunction including, but not limited to, changes that predispose to the development of non-insulin dependent diabetes.

Paradigms that involve systems in which the pancreatic islet or β-cell mass is manipulated may also be utilised. Such paradigms include pregnant animals and animals treated with agents that are known to produce an increase in pancreatic islet β-cell mass or proliferation of β-cells. Such agents include prolactin, glucagon-like peptide-1 and exendin-4.

In addition to whole animal studies, paradigms include systems in which isolated pancreatic islets or β-cells are incubated in vitro with agents that stimulate β-cell proliferation. Furthermore, the paradigm includes systems in which proliferation of β-cell lines such as Rin m5F, Rin 5F, HIT 15, MIN and BRINS cells is initiated in vitro.

### Analysis of paradigm material

In order to identify differentially expressed proteins, pancreatic islets and/or pancreatic β-cell from subjects utilised in paradigms such as those described above are obtained. Usually this will involve digestion of pancreatic tissue with enzymes such as collagenase. Methods for the isolation of pancreatic cells are well known to those in the art, as are methods of obtaining isolated β-cells from the islet tissue by such means as FACS sorting. In addition, blood and body fluids may be analysed since the differentially expressed proteins in pancreatic islets or β-cells might be released into the circulations.

Whole pancreatic islets, β-cell, and other islet cells may be used, as may whole pancreas including the exocrine tissue. Sub-cellular fractions of islets and isolated cells might also be used. Particularly useful sub-cellular fractions include the nuclear protein fraction.

### Characterisation of differentially expressed proteins

Differentially expressed proteins, such as those identified via the methods discussed above, as well as genes identified by alternative means, may be further characterised by utilising, for example, methods such as those discussed herein. Such proteins will be referred to herein as 'identified proteins'.

Analyses such as those described herein, yield information regarding the biological function of the identified proteins.

A variety of techniques can be utilised to further characterise the identified proteins. First, the corresponding nucleotide sequence of the identified protein may be obtained by utilising standard techniques well known to those of skill in the art, may, for example, be used to reveal homologies to one or more known sequence motifs which may yield information regarding the biological function of the identified protein.

Secondly, the biological function of the identified proteins may be more directly assessed by utilising relevant in vivo and in vitro systems. In vivo systems may include, but are not limited to, animal systems which naturally exhibit pancreatic islet and/or β-cell disorder-like symptoms, or ones which have been engineered to exhibit such symptoms. Further, such systems may include systems for the further characterisation of pancreatic islet and/or β-cell disorders, and/or non-insulin dependent diabetes, and may include, but are not limited to, naturally occurring and transgenic animal systems such as those described elsewhere in this specification. In vitro systems may include, but are not limited to, cell-based systems comprising cell types known to produce and secrete insulin. Such cells may be wild type cells, or may be non-wild type cells containing modifications known to, or suspected of, contributing to a pancreatic islet and/or β-cell disorder of interest. Such systems are discussed in detail below.

In further characterising the biological function of the identified proteins, the expression of these proteinss may be modulated within the in vivo and/or in vitro systems, i.e. either overexpressed or underexpressed in, for example, transgenic animals and/or cell lines, and its subsequent effect on the system then assayed. Alternatively, the activity of the identified protein may be modulated by either increasing or decreasing the level of activity in the in vivo and/or in vitro system of interest, and its subsequent effect then assayed.

The information obtained through such characterisations may suggest relevant methods for the treatment of pancreatic islet and/or β-cell disorders involving the protein of interest. Further, relevant methods for the control of non-insulin dependent diabetes involving the protein of interest may be suggested by information obtained from such characterisations. For example, treatment may include a modulation of protein expression and/or protein activity. Characterisation procedures such as those described herein may indicate where such modulation should involve an increase or a decrease in the expression or activity of the protein of interest. Such methods of treatment are discussed below.

### Cell- and animal-based model systems

Described herein are cell- and animal- based systems, which act as models for pancreatic islet and/or β-cell disorders. These systems may be used in a variety of applications. For example, the animal-based model systems can be utilised to identify differentially expressed proteins via one of the paradigms described above. Cell- and animal-based model systems may be used to further characterise differentially expressed proteins, as described above. Second, such assays may be utilised as part of screening strategies designed to identify compounds which are capable of ameliorating pancreatic islet and/or β-cell disorder symptoms, as described below. Thus, the animal- and cell-based models may be used to identify drugs, pharmaceuticals, therapies and interventions which may be effective in treating such pancreatic islet and/or β-cell disorders. In addition, such animal models may be used to determine the LD₅₀ and the ED₅₀ in animal subjects, and such data can be used to determine the in vivo efficacy of potential pancreatic islet and/or β-cell disorder treatments, including treatments for non-insulin diabetes.

### Animal-based systems

Animal-based model systems of pancreatic islet and/or β-cell disorders may include, but are not limited to, non-recombinant and engineered transgenic animals.

Non-recombinant animal models for pancreatic islet and/or β-cell disorders may include, for example, genetic models. Such-genetic pancreatic islet and/or β-cell models may include, for example, mouse models of non-insulin dependent diabetes and/or obesity such as mice homozygous for the autosomal recessive ob, db, or tub alleles. It could also include rat models, for example ZDF rats.

Non-recombinant, non-genetic animal models of pancreatic islet and/or β-cell disorder may include, for example, rats or mice fed on a diet containing a large amount of fat. Such diets could be synthetic diets in which the fat content (by calorific value) is more than 50%. Alternative human foods with a high fat content, such as salami and butter, may be provided to the animals. This model is particularly useful if it utilises low birth weight pups arising from a pregnant female fed on a low protein diet.

Additionally, animal models exhibiting pancreatic islet and/or β-cell disorder-like symptoms may be engineered by utilising, for example, the gene sequences of target proteins, in conjunction with techniques for producing transgenic animals that are well known to those of skill in the art. For example, gene sequences of target proteins may be introduced into, and overexpressed in, the genome of the animal of interest, or, if endogenous gene sequences of target proteins are present, they may either be overexpressed or, alternatively, may be disrupted in order to underexpress or inactivate gene expression of target proteins.

In order to overexpress the target gene sequence of a target protein, the coding portion of the target gene sequence may be ligated to a regulatory sequence, which is capable of driving gene expression in the animal and cell type of interest. Such regulatory regions will be well known to those of skill in the art, and may be utilised in the absence of undue experimentation.

For underexpression of an endogenous gene sequence of a target protein, such a sequence may be isolated and engineered such that when reintroduced into the genome of the animal of interest, the endogenous gene alleles of the target protein will be inactivated. Preferably, the engineered gene sequence of the target protein is introduced via gene targeting such that the endogenous sequence is disrupted upon integration of the engineered target gene sequence into the animal's genome.

Animals of any species, including, but not limited to, mice, rats, rabbits, guinea pigs, pigs, mini-pigs, goats and non-human primates, e.g. baboons, squirrels, monkeys and chimpanzees may be used to generate pancreatic islet and/or β-cell disorder animal models.

Any technique known in the art may be used to introduce a target gene transgene of a target protein into animals to produce the founder lines of transgenic animals. Such techniques include, but are not limited to, pronuclear microinjection (Hoppe, P.C. and Wagner, T.E., 1989, US Pat.No. 4,873,191); retrovirus mediated gene transfer into germ lines (Van der Putten et al., 1985, Proc. Natl. Acad. Sci., USA 82: 6148-6152); gene targeting in embryonic stem cells (Thompson et al., 1989, Cell 56: 313-321); electroporation of embryos (Lo, 1983, Mol. Cell Biol. 3: 1803-1819); and sperm-mediated gene transfer (Lavitrano et al., 1989, Cell 57: 717-723); etc. For a review of such techniques, see Gordon, 1989, Transgenic Animals, Intl. Rev. Cytol. 115: 171-229.

The present invention provides for transgenic animals that carry the transgene in all their cells, as well as animals which carry the transgene in some, but not all their cells, i.e. mosaic animals (see, for example, techniques described by Jakobovits, 1994, Curr. Biol. 4: 761-763). The transgene may be integrated as a single transgene or in concatamers, e.g. head-to-head tandems or head-to-tail tandems. The transgene may also be selectively introduced into and activated in a particular cell type by following, for example, the teaching of Lasko et al. (Lasko, M. et al., 1992, Proc. Natl. Acad. Sci. USA 89: 6232-6236). The regulatory sequences required to such a cell-type specific activation will depend upon the particular cell type of interest, and will be apparent to those of skill in the art.

When it is desired that the target gene transgene be integrated into the chromosomal site of the endogenous target gene, gene targeting is preferred. Briefly, when such a technique is to be utilised, vectors containing some nucleotide sequences homologous to the gene of the endogenous target protein of interest are designed for the purpose of integrating, via homologous recombination with chromosomal sequences, into and disrupting the function of, the nucleotide sequence of the endogenous target gene. The transgene may also be selectively introduced into a particular cell type, thus inactivating the endogenous gene of interest in only that cell type, by following, for example, the teaching of Gu et al. (Gu, H. et al., 1994, Science 265: 103-106). The regulatory sequences required for such a cell-type specific inactivation will depend upon the particular cell type of interest, and will be apparent to those of skill in the art.

Once transgenic animals have been generated, the expression of the recombinant target gene and protein may be assayed utilising standard techniques. Initial screening may be accomplished by Southern blot analysis or PCR techniques to analyse animal tissues to assay whether integration of the transgene has taken place.
The level of mRNA expression of the transgene in the tissues of the transgenic animals may also be assessed using techniques which include, but are not limited to, Northern blot analysis of tissue samples obtained from the animal, in situ hybridisation analysis, and RT-PCR. Samples of target protein-expressing tissue may also be evaluated immunocytochemically using antibodies specific for the transgene protein of interest.

The target protein transgenic animals that express target gene mRNA or target protein transgene peptide (detected immunocytochemically, using antibodies directed against target protein epitopes) at easily detectable levels should then be further evaluated to identify those animals which display characteristic pancreatic islet and/or β-cell disorder-like symptoms. Such symptoms may include, for example, obesity, glucose intolerance, hyperinsulinaemia, glycosuria and/or non-insulin dependent diabetes. Additionally, specific cell types within the transgenic animals may be analysed and assayed for cellular phenotypes characteristic of pancreatic islet and/or β-cell disorders. Further, such cellular phenotypes may include an assessment of a particular cell types fingerprint pattern of expression and its comparison to known fingerprint expression profiles of the particular cell type in animals exhibiting pancreatic islet and/or β-cell disorders. Such transgenic animals serve as suitable model systems for pancreatic islet and/or β-cell disorders.

Once target protein transgenic founder animals are produced (i.e. those animals which express target proteins in cells or tissues of interest and which, preferably, exhibit symptoms of pancreatic islet and/or β-cell disorders), they may be bred, inbred, outbred or crossbred to produce colonies of the particular animal. Examples of such breeding strategies include, but are not limited to, outbreeding of founder animals with more than one integration site in order to establish separate lines; inbreeding of separate lines in orde-r to produce compound target protein transgenics that transgenically express the target protein of interest at higher levels because of the effects of additive expression of each target gene transgene; crossing of heterozygous transgenic animals to produce animals homozygous for a given integration site in order to both augment expression and eliminate the possible need for screening of animals by DNA analysis; crossing of separate homozygous lines to produce compound heterozygous or homozygous lines; breeding animals to different inbred genetic backgrounds so as to examine effects of modifying alleles on expression of the target protein and the development of pancreatic islet and/or β-cell disorder-like symptoms. One such approach is to cross the target protein transgenic founder animals with a wild type strain to produce an F1 generation that exhibits pancreatic islet and/or β-cell disorder-like symptoms, such as glucose intolerance, hyperinsulinaemia, non-insulin dependent diabetes and obesity. The F1 generation may then be inbred in order to develop a homozygous line, if it is found that homozygous target protein transgenic animals are viable.

### Cell-based assays

Cells that contain and express target gene sequences which encode target proteins and, further, exhibit cellular phenotypes associated with an insulin resistance disorder, may be utilised to identify compounds that exhibit an ability to ameliorate pancreatic islet and/or β-cell disorder symptoms. Cellular phenotypes, which may indicate an ability to ameliorate pancreatic islet and/or β-cell disorders, may include, for example, abnormal insulin release in response to a glucose or glyceraldehyde stimulus.

Further, the fingerprint pattern of protein expression of cells of interest may be analysed and compared to the normal fingerprint pattern. Those compounds which cause cells exhibiting pancreatic islets and/or β-cell disorder-like cellular phenotypes to produce a fingerprint pattern more closely resembling a normal fingerprint pattern for the cell of interest may be considered candidates for further testing regarding an ability to ameliorate pancreatic islet and/or β-cell disorder symptoms.

Cells which can be utilised for such assays may, for example, include non-recombinant cell lines, such as Rin m5F, Rin 5F, Hit 15 and BRINS insulinoma cell lines.

Further, cell lines which may be used for such assays may also include recombinant, transgenic cell lines. For example, the pancreatic islet and/or β-cell disorder animal models of the invention discussed above may be used to generate cell lines, containing one or more cell types involved in pancreatic islet cell disorders, that can be used as cell culture models for this disorder. While primary cultures derived from the pancreatic islet cell disorder transgenic animals of the invention may be utilised, the generation of continuous cell lines is preferred. For examples of techniques which may be used to derive a continuous cell line from the transgenic animals, see Small, et al., 1985, Mol. Cell Biol. 5: 642-648.

Alternatively, cells of a cell type known to be involved in pancreatic islet cell disorders may be transfected with sequences capable of increasing or decreasing the amount of target protein within the cell. For example, gene sequences of target proteins may be introduced into, and overexpressed in, the genome of the cell of interest, or, if endogenous gene sequences of the target protein are present, they may either be overexpressed or, alternatively, be disrupted in order to underexpress or inactivate target protein expression.

In order to overexpress a gene sequence of a target protein, the coding portion of the target gene sequence may be ligated to a regulatory sequence, which is capable of driving gene expression in the cell type of interest. Such regulatory regions will be well known to those of skill in the art, and may be utilised in the absence of undue experimentation.

For underexpression of an endogenous target protein the gene sequence may be isolated and engineered such that when reintroduced into the genome of the cell type of interest, the endogenous target gene alleles will be inactivated. Preferably, the engineered target gene sequence is introduced via gene targeting such that the endogenous target sequence is disrupted upon integration of the engineered target gene sequence into the cell's genome.

Transfection of target protein gene sequence nucleic acid may be accomplished by utilising standard techniques. See, for example, Ausubel, 1989, supra. Transfected cells should be evaluated for the presence of the recombinant target gene sequences, for expression and accumulation of target gene mRNA, and for the presence of recombinant target protein production. In instances wherein a decrease in target protein expression is desired, standard techniques may be used to demonstrate whether a decrease in endogenous target gene expression and/or in target protein production is achieved.

### Diagnosis of pancreatic islet and/or β-cell disorder abnormalities including non-insulin dependent diabetes

The differentially expressed proteins may be employed for the diagnosis of pancreatic islet and/or β-cell disorders, the predisposition to pancreatic islet and/or β-cell disorders, and for monitoring the efficacy of any pancreatic islet and/or β-cell disorder compounds during, for example, clinical trials and for monitoring patients undergoing clinical evaluation for the treatment of such insulin resistance disorders. They can also be used to define the nature of a pancreatic islet or β-cell disorder to aid in the identification and/or selection of treatments for the disorder.

Conventional diagnostic methods include measurements of glucose/insulin ratios in whole animals or during glucose tolerance tests, c-peptide in urine or plasma.

Non-insulin dependent diabetes may be detected and the efficacy of treatment monitored by methods and parameters identified by such bodies as the World Health Organisation, the International Diabetes Federation and the American Diabetes Association.

Methods may, for example, utilise reagents such as antibodies directed against differentially expressed proteins. Specifically, such reagents may be used for (1) the detection of the presence of target protein mutations, or (2) the detection of either an over- or an under-abundance of target protein relative to the normal state.

Any cell type or tissue in which the differentially expressed protein is expressed may be utilised in the diagnostics described below. Examples of suitable samples types include cell samples, tissue samples, and fluid samples such as blood, urine or plasma.

The methods described herein may be used for monitoring the efficacy of compounds in clinical trials for the treatment of pancreatic islet and/or β-cell disorders. Such a method comprises detecting, in a patient sample, a protein, which is differentially expressed in the pancreatic islet and/or β-cell disorder state relative to its expression in a normal state, as set out in the claims.

During clinical trials, for example, the expression of a single differentially expressed protein, or alternatively, a fingerprint pattern of a cell involved in a pancreatic islet and/or β-cell disorder can be determined in the presence or absence of the compound being tested. The efficacy of the compound can be followed by comparing the expression data obtained to the corresponding known expression patterns in a normal state. Compounds exhibiting efficacy are those which alter the single fingerprint protein expression and/or the fingerprint pattern to more closely resemble that of the normal state.

The detection of the protein differentially expressed in a pancreatic islet and/or β-cell disorder state relative to their expression in a normal state can also be used for monitoring the efficacy of potential pancreatic islet and/or β-cell disorder compounds and compounds for the treatment of non-insulin dependent diabetes during clinical trials. During clinical trials, for example, the level and/or activity of the differentially expressed protein can be determined in relevant cells and/or tissues in the presence or absence of the compound being tested. The efficacy of the compound can be followed by comparing the protein level and/or activity data obtained to the corresponding known levels/activities for the cells and/or tissues in a normal state. Compounds exhibiting efficacy are those which alter the pattern of the cell and/or tissue involved in the pancreatic islet and/or β-cell disorder to more closely resemble that of the normal state.

### Example 1:

### Mouse treatment protocol

Lean and obese female C57 B1/6J ob/ob mice were given BRL 49653, rosiglitazone, 10mg/kg/day, by oral gavage for 7 days. This treatment produced significant improvement in oral glucose tolerance and insulin sensitivity in ob/ob mice, but had no effect in the lean litter mates. Non-fasting animals were anaesthetised with 50% Hyponovel Trade Mark and 50% Hyponorm Trade Mark and then killed humanely with carbon dioxide gas. The pancreas was then removed.

### Preparation of pancreatic islets

Pancreatic islets were isolated by collagenase digestion (collagenase type VI, Sigma, UK) at 37°C using a physiological saline solution supplemented with 1mM CaCl₂, 4mM glucose and equilibrated with CO₂:O₂ (5%:95%), pH 7.4. Islets were hand-picked using a binocular microscope.
The islets were washed and further microdissected in order to remove residual acinar material.

The islets were then snap frozen in liquid N₂ and stored frozen.

### Protein solubilisation

For analytical 2-D-PAGE, 125 pancreatic islets were mixed with 60 microlitres of a solution containing urea (8M), CHAPS (4% w/v), Tris (40mM), DTE (65mM), SDS (0.05% w/v) and a trace of bromophenol blue. A weighed portion of the final diluted sample was loaded into a sample cup at the cathodic end of the IPG gels.

### First dimension electrophoresis

A non-linear immobilised pH gradient of IPG strips (3.5-10.0 NL IPG 18cm) was used as the first dimension. It offered high resolution, great reproducibility and allowed high protein loads. Based on specifications of the Geneva University Hospital, the non-linear pH gradient strips were prepared by Amersham-Pharmacia Biotechnology AB and are commercially available. The strips were 3mm wide and 180mm long.

Hydration of the IPG strips was performed overnight in a Pharmacia reswelling cassette with 25ml of a solution of urea (8M), CHAPS (2% w/v), DTE (10mM), Resolyte pH 3.5-10 (2% v/v) and a trace of bromophenol blue.

When the rehydration cassette had been thoroughly emptied and opened, the strips were transferred to the Pharmacia strip tray. After placing IPG strips, humid electrode wicks, electrodes and sample cups in position, the strips and cups were covered with low viscosity paraffin oil. Samples were applied in the cups at the cathodic end of the IPG strips in a slow and continuous manner, without touching the gel.

The voltage was linearly increased from 300 to 3500 V during 3 hours, followed by 3 additional hours at 3500 V, whereupon the voltage was increased to 5000 V. A total volt hour product of 100kvh was used in an overnight run.

### Second dimension of the electrophoresis

After the first dimension run, the IPG strips were equilibrated in order to resolubilise the proteins and to reduce -S-S- bonds. The strips were thus equilibrated within the strip tray with 100ml of a solution containing Tris-HCl (50mM), pH 6.8, urea (6M), glycerol (30% v/v), SDS (2% w/v) and DTE (2% w/v) for 12 min. The SH groups were subsequently blocked with 100 ml of a solution containing Tris-HCl (50mM) pH 6.8, urea (6M), glycerol (30% v/v), SDS (2% w/v), iodoacetamide (2.5% w/v) and a trace of bromophenol blue for 5 min.

In the second dimension run, a vertical gradient slab gel with the Laemmli-SDS-discontinuous system was used with some small modifications, which may be summarised as follows:
- Gels are not polymerised in the presence of SDS. This seems to prevent the formation of micelles, which contain acrylamide monomer, thus increasing the homogeneity of pore size and reducing the concentration of unpolymerised monomer in the polyacrylamide. The SDS used in the gel running buffer is sufficient to maintain the necessary negative charge on proteins.
- Piperazine-diacrylyl (PDA) is used as crosslinker. This is believed to reduce N-terminal protein blockage, gives better protein resolution, and reduces diammine silver staining background.
- Sodium thiosulphate is used as an additive to reduce background in the silver staining of gels.
- The combination of the IPG strip and agarose avoids the need for a stacking gel.

The gel composition and dimensions were as follows:

| | |
|---|---|
| Dimensions: | 160 x 200 x 1.5mm |
| Resolving gel: | Acrylamide/PDA (9-16% T/2.6% C) |
| Stacking gel: | No stacking |
| Leading buffer: | Tris-HCl (0.375M) pH 8.8 |
| Trailing buffer: | Tris-glycine-SDS (25mM-198mM-0.1% w/v) pH 8.3 |
| Additives: | Sodium thiosulphate (5mM) |
| Polymerisation agents: | TEMED (0.05%) APS (0.1%) |

The gels were poured until 0.7cm from the top of the plates and over-layered with sec-butanol for about two hours. After the removal of the overlay and its replacement with water the gels were left overnight.

After the equilibration, the IPG gel strips were cut to size. Six mm were removed from the anodic end and 14mm from the cathodic end. The second dimension gels were over-layered with a solution containing agarose (0.5% w/v) and Tris-glycine-SDS (25mM-198mM-0.1% w/v) pH 8.3 heated at about 70°C and the IPG gel strips were immediately loaded through it.

The gel was run at 8-12°C for 5 hours at a constant current of 40mA/gel. The voltage is non-limiting, but usually requires 100 to 400 V.

### Staining

Silver staining, which is 100-fold more sensitive than Coomassie Brilliant Blue staining, was used (except where otherwise stated). Thus, the 2-DGE gels were stained with an ammoniacal silver staining as follows:
All steps were performed on an orbital shaker at 36 rpm.
Step 1: At the end of the second dimension run, the gels were removed from the glass plates and washed in deionised water for 5 min.
Step 2: The gels were soaked in ethanol:acetic acid:water (40:10:50 volume ratio) for 1 hour.
Step 3: The gels were soaked in ethanol:acetic acid:water (5:5:90 volume ratio) for 2 hours or overnight.
Step 4: They were washed in deionised water for 5 min.
Step 5: They were soaked in a solution containing glutaraldehyde (1% w/v and sodium acetate (0.5M) for 30 min.
Step 6: They were washed 3 times in deionised water for 10 min.
Step 7: In order to obtain homogenous dark brown staining of proteins, gels were soaked twice in a 2, 7-naphthalenedisulphonic acid solution (0.05% w/v) for 30 min.
Step 8: The gels were then rinsed 4 times in deionised water for 15 min.
Step 9: The gels were stained in a freshly made ammoniacal silver nitrate solution for 30 minutes. To prepare 750ml of this solution, 6g of silver nitrate were dissolved in 30ml of deionised water, which was slowly mixed into a solution containing 160ml of water, 10ml of concentrated ammonia (25%) and 1.5ml o sodium hydroxide (10N). A transient brown precipitate might form. After it cleared, water was added to give the final volume.
Step 10: After staining, the gels were washed 4 times in deionised water for 4 min.
Step 11: The images were developed in a solution of citric acid (0.01% w/v) and formaldehyde (0.1% v/v) for 5 to 10 min.
Step 12: When a slight background stain appeared, development was stopped with a solution of Tris (5% w/v) and acetic acid (2% v/v).

### Scanning of the gels

The Laser Densitometer (4000 x 5000 pixels; 12 bits/pixel) from Molecular Dynamics and the GS-700 from Bio-Rad have been used as scanning devices. These scanners were linked to Sparc Trade Mark workstations and Macintosh Trade Mark computers.

### Quantitative image analysis of the gels using Melanie II Trade Mark

Two-dimensional polyacrylamide gels may be digitised and analysed by computer to allow quantitative image analysis and automatic gel comparison. Since the 2-D-PAGE technique was first developed in 1975 several computer systems have been manufactured, mainly by academic 2-D-PAGE related laboratories. In the present work, Melanie II Trade Mark, developed at the University Hospital of Geneva was used. It is available for Unix Trade Mark workstations, as well as for Power Macintosh Trade Mark and IBM-compatible computers.

ob/ob and lean mice spot detection, quantitation and matching, gel image extraction, zooming, warping and printing as well as gel stacking and flipping were carried out with the MelView Trade Mark program.

The images were then classified in four classes; lean control, ob/ob control, lean treated and ob/ob treated. Differential analysis and the Student T test, using the relative abundance of each spot (% volume), allowed the detection of significant (p<0.01) over- and underexpressed polypeptides.

### Preparative 2-D-PAGE

The analytical 2-DGE described above were repeated, using 1000-2000 islets. After the first dimension run the strips were equilibrated using 3ml of each buffer per groove.

### Protein electroblotting

The blotting of proteins separated by 2-D-PAGE onto polyvinylidene difluoride (PVDF) membranes has enabled the identification and characterisation of proteins from complex biological samples. Transfer of the proteins can be carried out using several methods such as vacuum, capillary or electric field. Electroblotting, using vertical buffer tanks or a semi-dry method, is preferred. Both techniques can use the 3-[cyclohexamino]-1-propanesulfonic acid (CAPS) transfer buffer. Gloves must be worn and all filter papers should be washed three times for 3 min in water three times in transfer buffer. These two steps are important in order to avoid any protein or amino acid contamination.

The procedure was as follows. After second dimensional electrophoresis, the gels were soaked in deionised water for 3 min. Then they were equilibrated in a solution containing 10mM CAPS pH 11 for 30 min. At the same time, PVDF membranes were wetted in methanol for 1 min and equilibrated in a solution containing 10mM CAPS pH 11 and methanol (10% v/v) also for 30 min. Electroblotting was carried out in a semi-dry apparatus with a solution containing 10mM CAPS pH 11 and methanol (20% v/v anodic side; 5% v/v cathodic side) at 1 mA/cm² constant current for 3 hours at 15°C.

### Protein detection on PVDF membranes

Amido Black and Coomassie Brilliant Blue R-250 were used instead of silver staining to visualise proteins on PVDF membranes and are compatible with the ensuing post-separation analysis. Thus, in another 2-DGE run, after electrotransfer, the PVDF membranes were stained in a solution containing Amido Black (0.5% w/v, isopropanol (25% v/v) and acetic acid (10% v/v) for 2 min. Destaining was done by several soakings in deionised water.

In another run, after electrotransfer, the PVDF membranes were stained in a solution containing Coomassie Brilliant Blue R-250 (0.1% w/v) and methanol (50% v/v) for 15 min. Destaining was done in a solution containing methanol (40% v/v) and acetic acid (10% v/v). The same method was used for preparative gels that did not need electrotransfer for further post-separation analysis, such as peptide mass fingerprinting.

The PVDF stained membranes were either air-dried or dried on a 3mm thick plate onto a heating plate at 37°C for 10 min.

### Scanning

This was done as described above.

### Protein identification

In amino sequence analysis by Edman degradation, amino acid derivatives are sequentially cleaved one at a time from the protein. Proteins with a chemically inaccessible alpha-amino group cannot be sequenced directly by this procedure and are termed N-terminally blocked. The best way to overcome the blocked proteins is to generate individual fragments by chemical or proteolytic cleavage. Routinely, ten to twelve Edman degradation cycles were performed for each spot. A search in the SWISS-PROT database was made to detect identity to known protein sequences.

The Amido Black stained proteins were excised with a razor blade and N-terminal sequencing was performed using an ABI model 473A or 477A microsequencer from Applied Biosystems equipped with "Problott" cartridges.

For internal sequencing, the spots of interest were excised and soaked for two hours in a solution containing acetic acid (100mM), methanol (10% v/v) and PVP-40 (1% v/v) at 37°C. After three washes in deionised water, the PVDF spots were cut into small pieces (about 1 mm²) and incubated in 25 microlitres of a solution containing sodium phosphate (100mM) pH 8.0 and lysyl endopeptidase (1 microgram). Following overnight digestion a room temperature, guanidine-HCl (28mg) and DTT (100 micrograms) were added. After reduction for 2 hours at 37°C, the mixture was incubated for 30 min, at room temperature, with 300 micrograms of iodoacetamide. The digestion solution was removed and kept. PVDF pieces were then extracted overnight with 25 microlitres of a solution containing isopropanol (70% v/v) and trifluoroacetic acid (5% w/v). This elution solution was removed and the PVDF was washed twice with 60 microlitres of TFA (0.1% w/v). The digestion and elution solutions were pooled together with two final washes and this mixture was separated by two-dimensional reverse phase HPLC and sequence determination performed.

### Immunoblotting

PVDF membranes were first stained to visualise proteins, following which the immunodetection was undertaken. This allowed matching of proteins detected with ECL against those detected with the non-specific protein stain through computer comparison of both images. The mechanical strength of PVDF was also exploited as the same 2-D gel can be used many times for different antibodies.

The whole procedure was carried out in a rotating oven at room temperature. The use of a nucleic acid glass hybridiser tube minimised the volumes and costs.
- The membranes were blocked in 10ml of a solution of PBS (pH 7.2) and non-fat dry milk (5% w/v) for 30 min.
- The membranes were then incubated in 10ml of a solution containing PBS- Tween Trade Mark 20 (0.5% v/v), non-fat dry milk (5% w/v) and the primary antibody/antibodies (1:100 or greater, depending on the antibody) for 2 hours.
- Three quick rinses were performed with 10 ml of PBS-Tween Trade Mark 20 (0.5% v/v) and then the membranes were washed for 3 x 10 min with 10ml of PBS- Tween Trade Mark 20 (0.5% v/v).
- The membranes were incubated in 10ml of a solution containing PBS- Tween Trade Mark 20 (0.5% v/v|), non-fat dry milk (5% w/v) and the secondary peroxidase-conjugated antibody (1:1000; for example, if the primary antibody was sheep anti-mouse, then goat anti-sheep IgG was used as the secondary antibody) for 1 hour.
- Three quick rinses were performed with 10ml of PBS-Tween Trade Mark 20 (0.5% v/v) and then the membranes were washed for 5 x 10 min with 10ml of PBS- Tween Trade Mark 20 (0.5% v/v).
- After the last wash, the membranes were transferred to a clean glass plate and covered with 10ml of developing solution (for example ECL from Amersham International or Roche Diagnostics) for 2 min.
- The excess developing solution was drained, the membranes were wrapped in Saran Trade Mark film and fixed in an X-ray film cassette with the proteins facing up.
- An X-ray film was then exposed in a dark room for a few seconds or up to several minutes.

### Peptide mass fingerprinting

The 2-DGE method was repeated, but using a Coomassie blue stain. The 2-DGE spots were destained with 100 microlitres of 30% acetonitrile in 50mM ammonium bicarbonate at 37°C for 45 min. The supernatant was discarded and the gel spots dried in a SpeedVac Trade Mark for 30min. The gel spots were rehydrated with 25 microlitres of a solution containing 0.2 micrograms of porcine trypsin and 50mM ammonium bicarbonate for 2 hours at 35°C and dried again for 30 min. Twenty microlitres of a solution of 50% of acetonitrile and 0.1% of TFA was added to the spots and sonicated for 10 min. Two microlitres of the supernatants was loaded in each well of a 96 or 400 MALDI target plate. The samples were air-dried. Then 2 microlitres of a solution containing 4mg/ml of alpha-cyano-4-hydroxycinnamic acid, 50% acetonitrile and 0.1% TFA was added to each well and air-dried.

The peptide mixtures were analysed by matrix-assisted laser desorption/ionisation time-of-flight mass spectrometer (Perseptive Biosystems Voyager Elite MALDI-TOF-MS) with a nitrogen laser (337nm) and operated in reflectron delayed extraction mode.

Peptide identification has been carried out using PeptIdent Trade Mark (http://www.expasy.ch/sprot/peptident.html). It is a tool that allows the identification of proteins using pI, relative molecular mass and peptide mass fingerprinting data. Experimentally measured, user-specified peptide masses were compared with the theoretical peptides calculated for all proteins in the SWISS-PROT/TREMBL databases.

### MS/MS sequencing

When protein identification was not successful with the peptide mass fingerprinting procedure, the supernatant of digested spots was desalted in ZipTip Trade Mark C18 pipette tips (Millipore) and eluted with 50% acetonitrile and 0.1% TFA. Peptides were applied by nanoflow (in-house nanospray) sample introduction to a tandem mass spectrometer that consists of two quadrupoles and an orthogonal time of flight tube (Q-TOF) from MicroMass (UK). Fragment ion spectra were interpreted with the MOWSE database search (http://www.seqnet.dl.ac.uk/mowse.html).

### Data management: the mouse SWISS-2-DPAGE database

SWISS-2-DPAGE is an annotated 2-D-PAGE database in which all the data are easily retrieved by computer programs and stored in a format similar to that of the SWISS-PROT Protein Sequence Database, one of the most updated and annotated protein sequence databases presently available. The SWISS-2-DPAGE database assembles data on proteins identified on various 2-D-PAGE maps. Each SWISS-2-DPAGE entry contains data on one protein, including mapping procedures, physiological and pathological data and bibliographical references, as well as several 2-D-PAGE images showing the protein location. Cross-references are provided to SWISS-PROT and, through the latter, to other databases (EMBL, Genbank, PROSITE, OMIM, etc.). The database has been set upon the ExPASy World Wide Web server (http://www.expasy.ch/). Worldwide, scientists using similar 2-D-PAGE protocols (immobilised pH gradient as first dimensional separation) are now able to compare their images with SWISS-2-DPAGE maps.

### Results

The following DEPs were found:
Group 1. POM6, POM7, POM8, POM9 and POM10 all had a reduced expression in islets from ob/ob control mice relative to their expression in islets from lean control mice. Their expression in ob/ob islets was not increased by treatment with rosiglitazone.
Group 2. POM (T) 1, POM(T)2, POM (T) 4 all had a reduced expression in islets from ob/ob control mice relative to the expression in lean control mice and their expression in ob/ob mice were increased towards the level in lean control mice following treatment with rosiglitazone.

POM(T)3, POM(T)5, POM(T)11, POM(T)12 and POM(T)13 all had an increased expression in islets from ob/ob control mice relative to the level in lean control mice and treatment with rosiglitazone decreased their expression towards that in lean control mice. Thus treatment of ob/ob mice with rosiglitazone, which improves diabetic control in these mice, restored the expression of these proteins in ob/ob mice towards the expression level in lean mice. In each case, similar treatment of lean mice with rosiglitazone had no effect on the expression level and therefore the alteration in pancreatic islet protein expression appears to be related to improved pancreatic islet function associated with the rosiglitazone treatment.

Group 3. Two proteins PSEM14 and PSEM15 did not show any differential expression between lean control and ob/ob control mice but treatment with rosiglitazone increased the expression of these proteins in both lean and ob/ob mice.

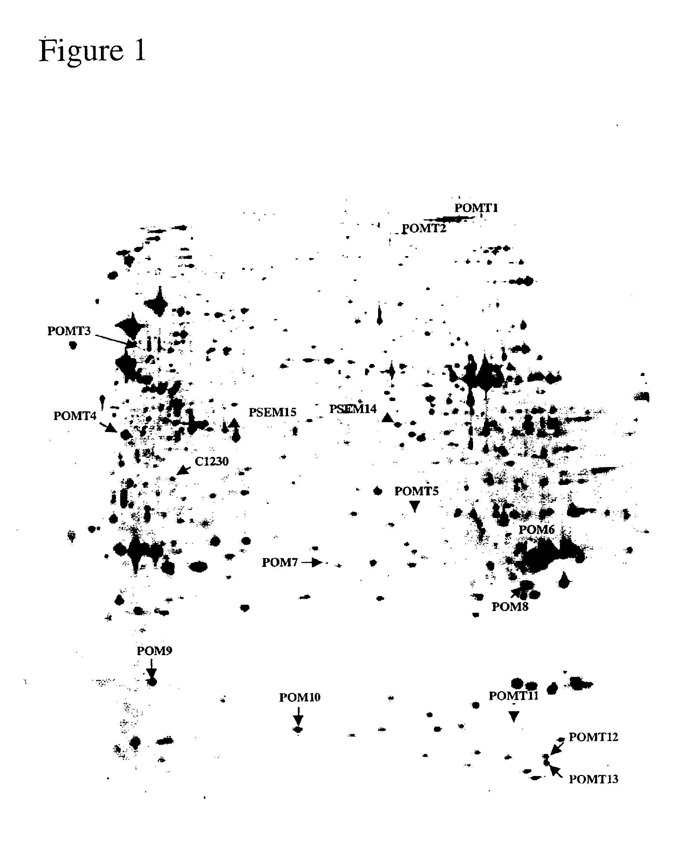

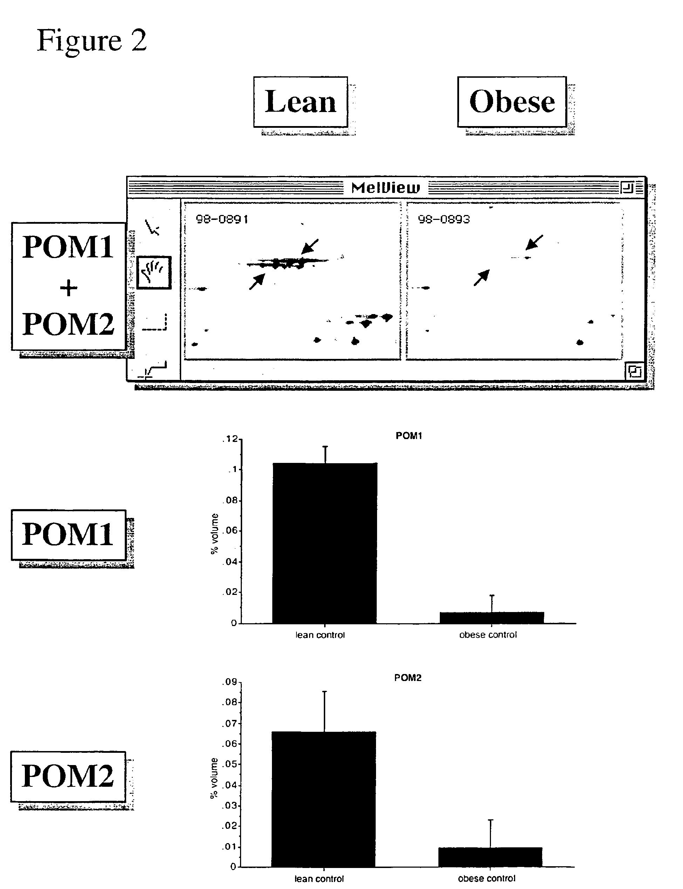

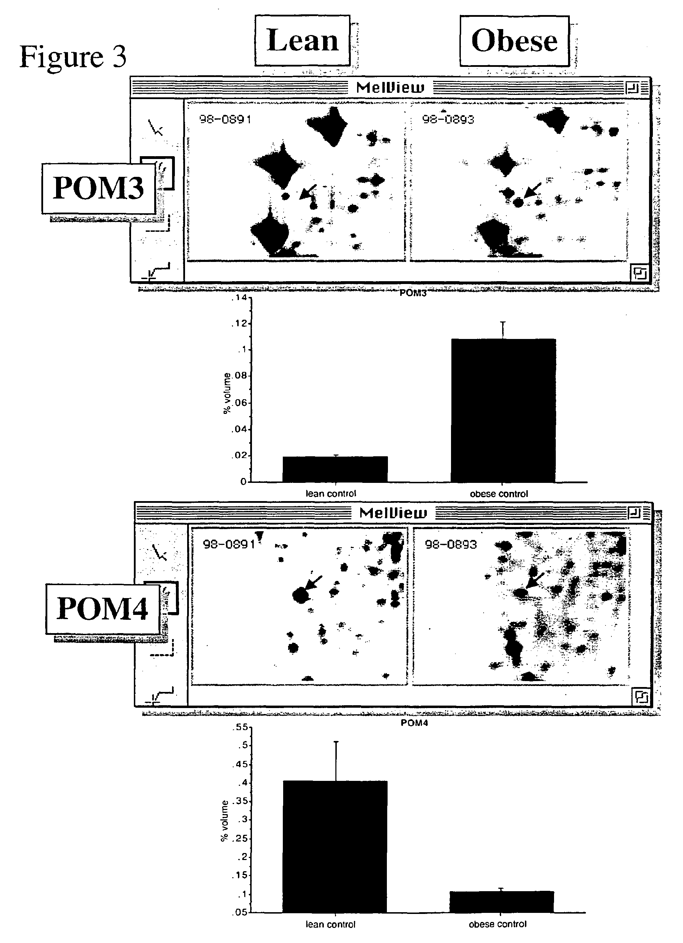

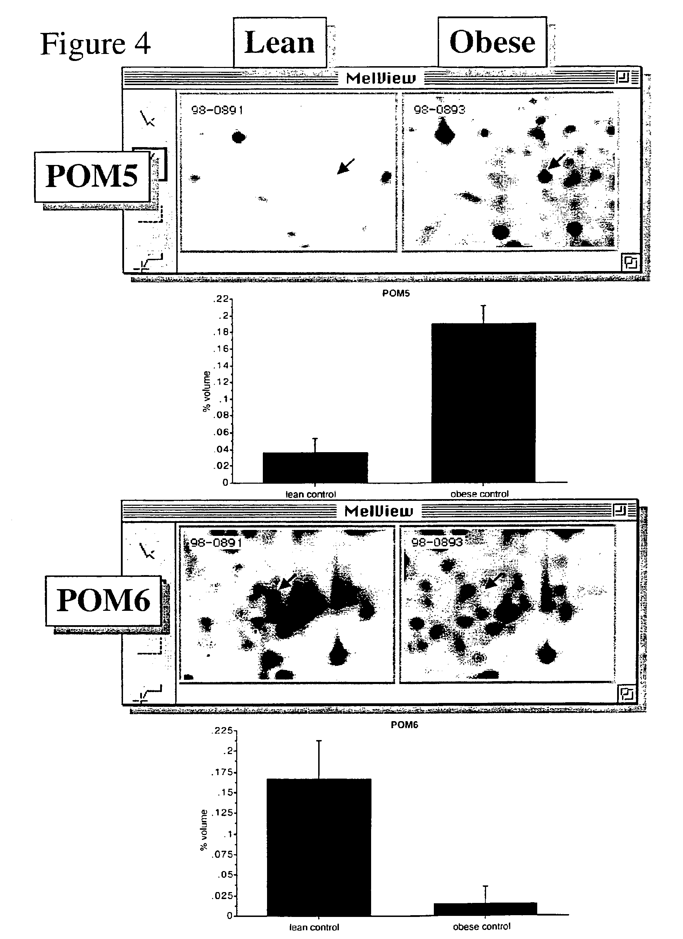

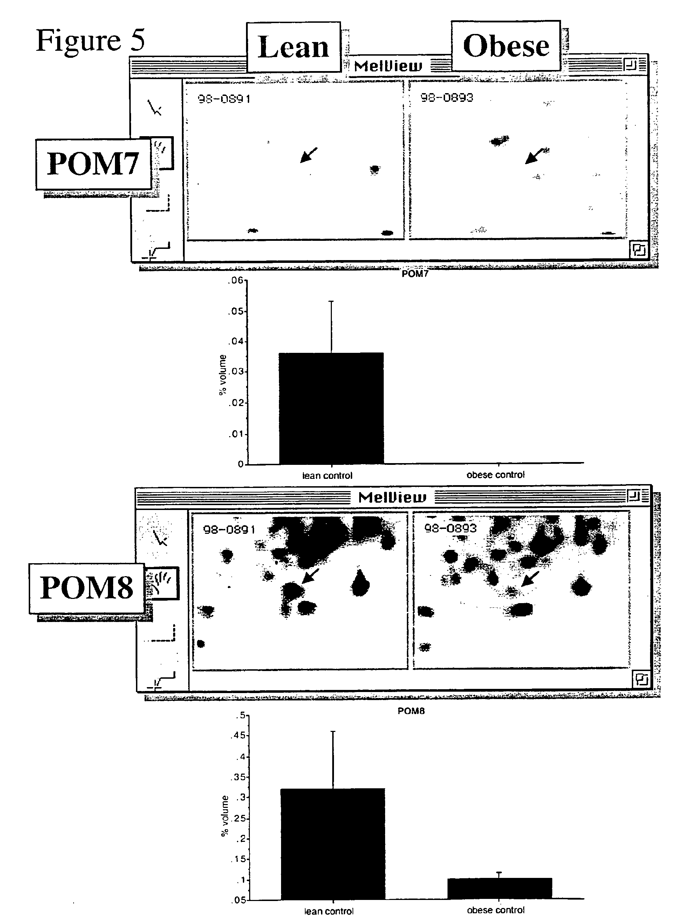

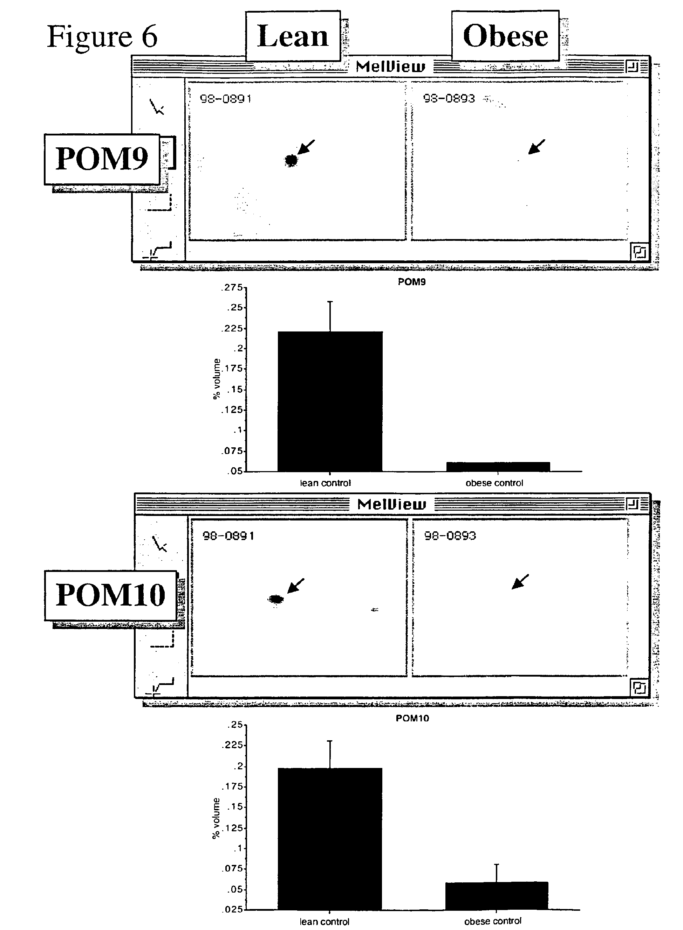

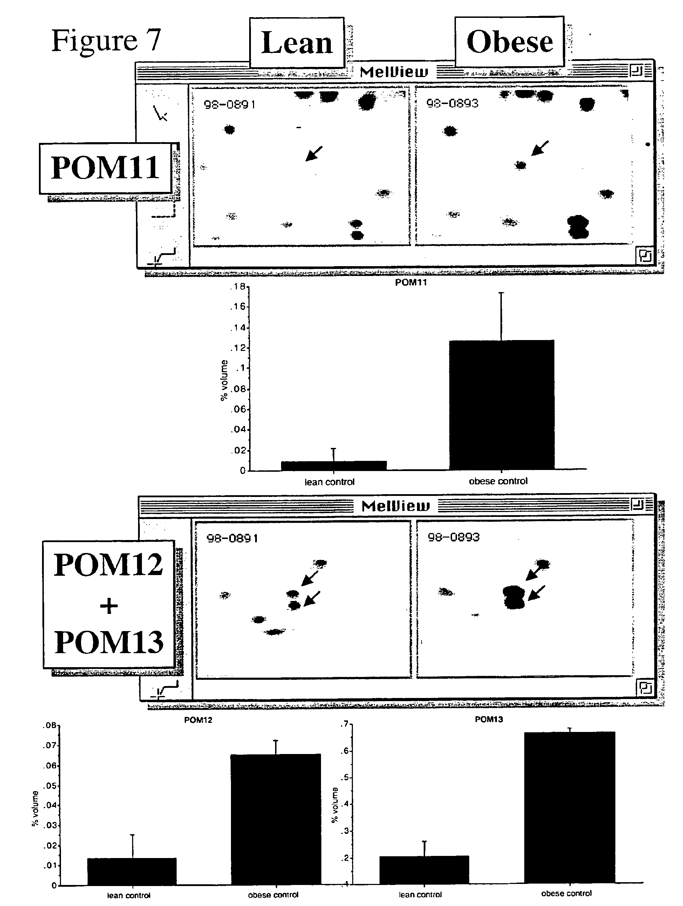

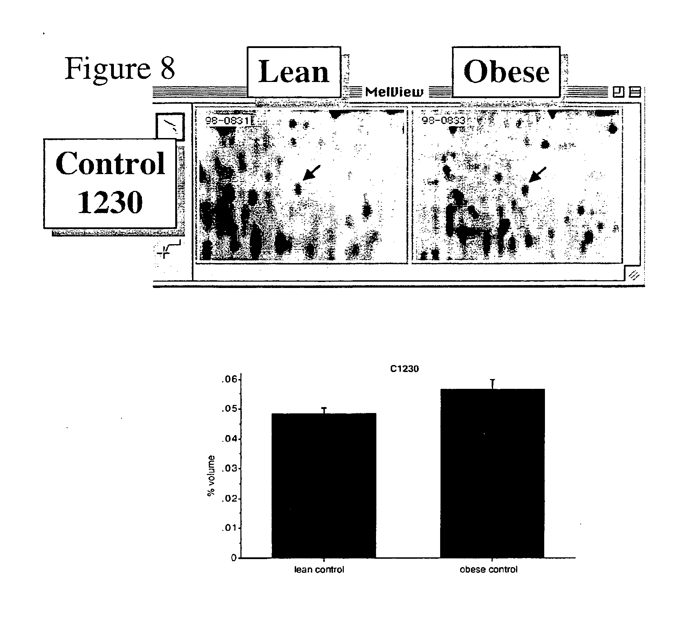

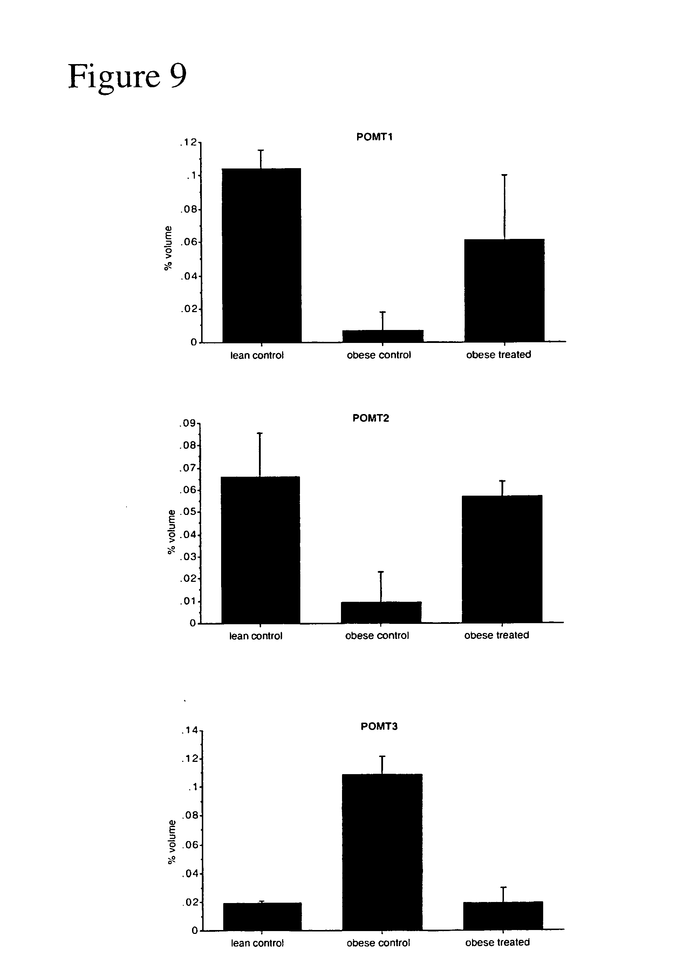

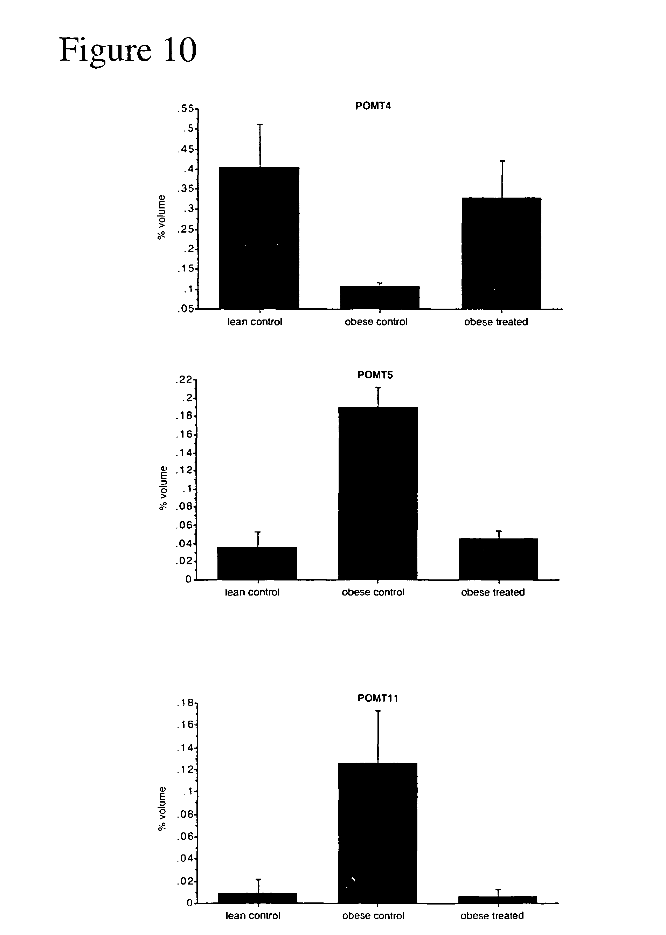

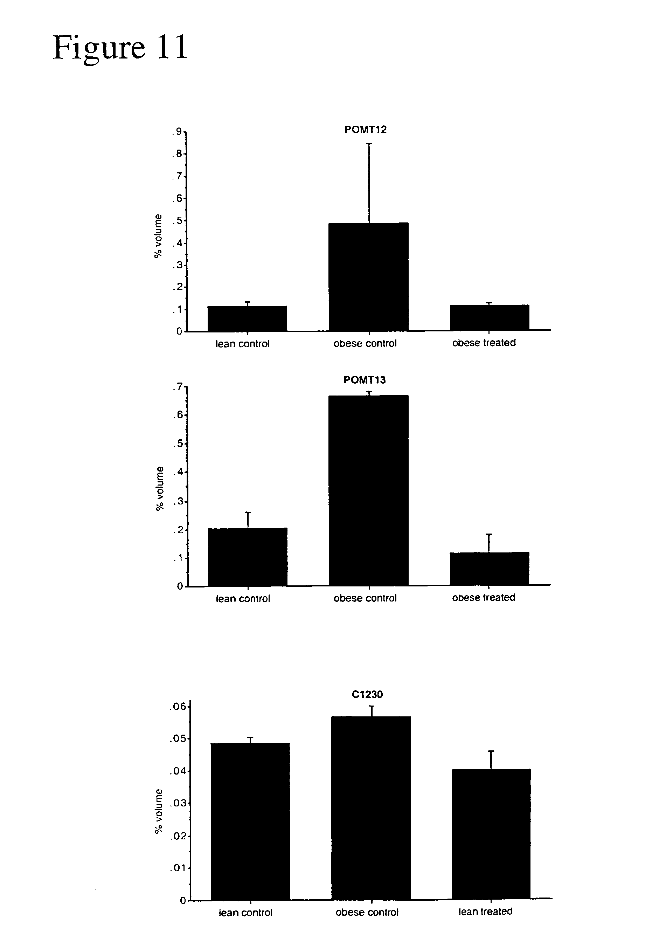

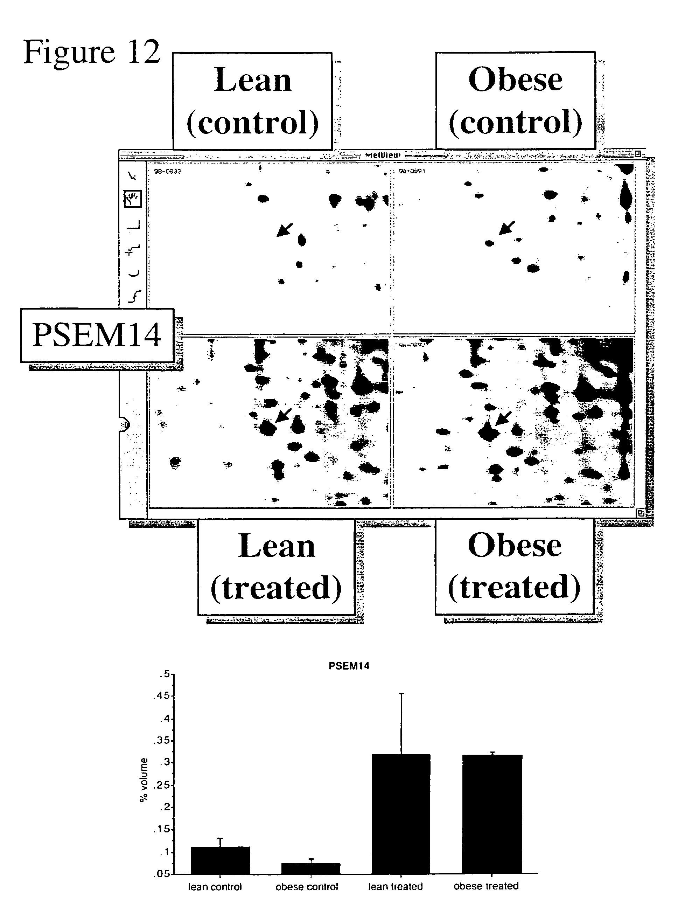

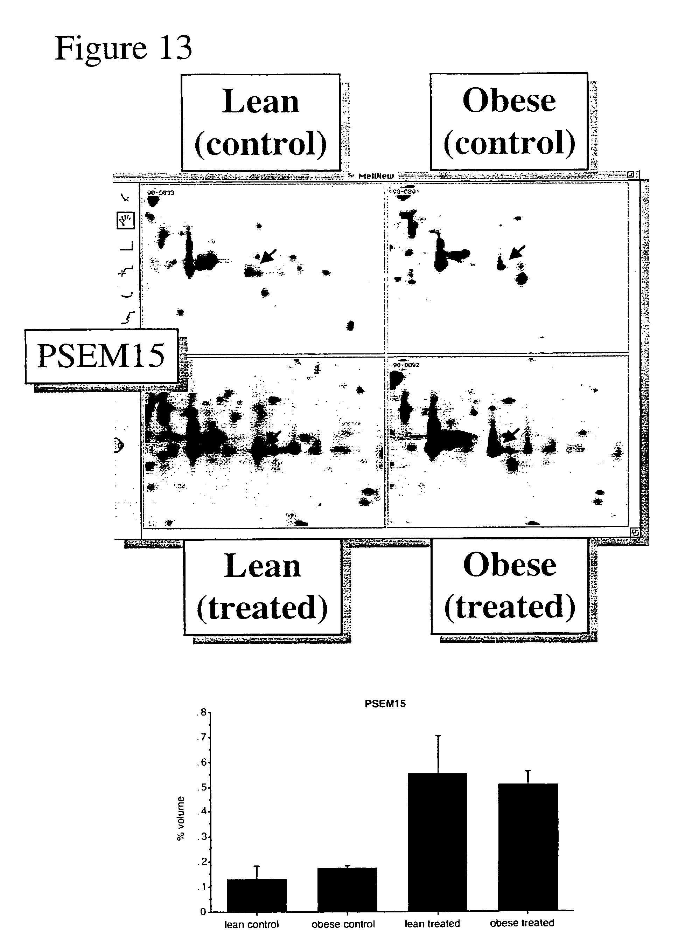

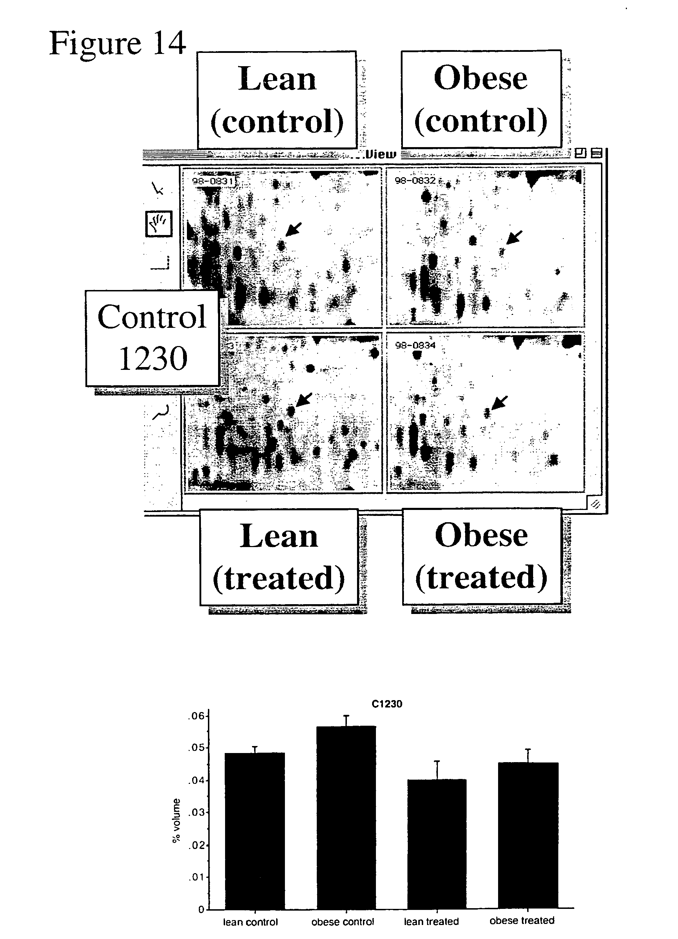

The drawings are presented to show the location of the DEPs. Fig. 1 shows the 2D maps of proteins from the pancreatic islets of lean control mice. Figs. 2-7 and Figs. 9-13 show the comparative expression of the DEPs together with quantification of the differential expression. With comparative purposes, Figs. 8 and 14 show a control protein (C1230) that is not differentially expressed.

**Table 1:**

| MARKER | GENE NAME | PROTEIN DESCRIPTION | SWISS-PROT AC | METHOD OF ID. |
|---|---|---|---|---|
| POMT1 | - | - | - | - |
| POMT2 | - | - | - | - |
| POMT3 | - | - | - | - |
| POMT4 | TM1 | Tropomyosin isoform 1 | P46901 | MS/MS |
| POMT5 | - | - | - | - |
| POM6 | DHPR | Dihydropteridine reductase | P11348 | MS/MS |
| POM7 | DHPR | Dihydropteridine reductase fragment | P11348 | MS/MS |
| POM8 | GSTP2 | Glutathione S-transferase P1 | P19157 | MS/MS |
| POM9 | CHGA | Chromogranin A (peptide 19-151 Beta grain) | P26339 | MS/MS |
| POM 10 | S100A9 | Calgranulin B | P06702 | MS/MS |
| POMT11 | FABP4 | Fatty acid binding protein, adipocyte | P04117 | MS/MS |
| POMT12 | PFN1 | Profilin | P10924 | MS/MS |
| POMT13 | PFN1 | Profilin (fragment) | P10924 | MS/MS |
| PSEM14 | - | - | - | - |
| PSEM15 | CPB | Carboxypeptidase B (with propeptide) | P19223 | MS/MS |

**Table 2:**

| PROTEIN | VOL | %VOL | AREA | %OD | OD | pl | Mw |
|---|---|---|---|---|---|---|---|
| POMT1 | 0.046 | 0.014 | 0.827 | 0.217 | 0.286 | 6.17 | 186623 |
| POMT2 | 0.144 | 0.043 | 1.041 | 0.582 | 0.767 | 6.01 | 180774 |
| POMT3 | 0.086 | 0.026 | 0.919 | 0.356 | 0.469 | 5.01 | 63585 |
| PSEM15 | 2.777 | 0.837 | 5.911 | 1.9 | 2.506 | 5.28 | 44751 |
| PSEM14 | 0.156 | 0.047 | 0.919 | 0.776 | 1.024 | 6.1 | 45123 |
| POMT4 | 0.367 | 0.111 | 1.225 | 1.196 | 1.577 | 4.98 | 40622 |
| C1230 | 0.068 | 0.021 | 0.98 | 0.247 | 0.326 | 5.06 | 36917 |
| POMT5 | 0.154 | 0.046 | 1.072 | 0.603 | 0.795 | 6.33 | 30681 |
| POM6 | 1.115 | 0.336 | 2.726 | 1.556 | 2.052 | 7.11 | 26798 |
| POM7 | 0.146 | 0.044 | 1.194 | 0.508 | 0.67 | 5.79 | 25939 |
| POM8 | 0.743 | 0.224 | 2.45 | 1.147 | 1.513 | 7.05 | 23525 |
| POM9 | 0.067 | 0.02 | 1.133 | 0.21 | 0.277 | 5.03 | 18905 |
| POM10 | 0.102 | 0.031 | 1.378 | 0.301 | 0.397 | 5.53 | 15637 |
| POMT11 | 0.213 | 0.064 | 1.531 | 0.572 | 0.754 | 6.86 | 14446 |
| POMT12 | 0.6 | 0.181 | 1.776 | 1.351 | 1.782 | 7.43 | 13828 |
| POMT13 | 2.425 | 0.732 | 5.206 | 1.926 | 2.54 | 7.42 | 13402 |

## Claims

1. A method of screening a test agent to determine its usefulness in treating a condition **characterised by** pancreatic islet or β-cell dysfunction, the method comprising:
(a) establishing a paradigm in which at least one protein is differentially expressed in relevant tissue from, or representative of, subjects having differential levels of pancreatic islet or β-cell function, the subjects comprising:
(i) normal subjects who have and have not been treated with an agent which improves pancreatic islet or β-cell function, and
(ii) affected subjects having reduced or increased pancreatic islet or β-cell function relative to the normal subjects, wherein said affected subjects have and have not been treated with the agent which improves pancreatic islet or β-cell function,
wherein said differentially expressed protein is differentially expressed between said affected and normal subjects who have not been treated with the agent which improves pancreatic islet or β-cell function, and is differentially expressed between said affected subjects who have and have not been treated with the agent which improves pancreatic islet or β-cell function, but is not differentially expressed between said normal subjects who have and have not been treated with the agent which improves pancreatic islet or β-cell function;
(b) providing a sample of relevant tissue which has been obtained from, or is representative of, a test subject having reduced pancreatic islet or β-cell function, who or which has been treated with the test agent being screened;
(c) determining the presence, absence or degree of expression of the differentially expressed protein or proteins in the tissue from, or representative of, the treated test subject; and,
(d) selecting or rejecting the agent according to the extent to which it changes the expression, activity or amount of the differentially expressed protein or proteins in the treated test subject having reduced pancreatic islet or β-cell function.

2. The method of claim 1, wherein the test agent is selected if it converts the expression of the differentially expressed protein or proteins towards that of a subject having more normal pancreatic islet or β-cell function.

3. The method of claim 1 or claim 2, wherein the test agent is selected if it converts the expression of the protein or proteins to that of the normal subject.

4. The method of any one of claims 1 to 3, wherein the pancreatic islet or β-cell dysfunction is a result of a disorder which causes a reduction in pancreatic islet or β-cell mass and/or a reduction in a pancreatic islet or β-cell biological activity.

5. The method of any one of the preceding claims, wherein the paradigm is based on tissue from non-insulin dependent diabetic subjects and normal subjects.

6. The method of any one of the preceding claims wherein the relevant tissue is pancreatic islets.

7. The method of claim 1, wherein the paradigm is based on:
(a) animals which are models of non-insulin dependent diabetes as a result of a genetic mutation such as ob/ob, db/db, agouti, fat, fa/fa together with lean littermates; or
(b) animals in which islet or β-cell dysfunction is exacerbated by dietary treatment; or
(c) the offspring of pregnant animals fed on a reduced protein diet, wherein optionally the diet fed to the offspring post weaning is additionally a high fat diet; or
(d) desert rodents such as spiny mice or sand rats which develop diabetes on normal laboratory diets but remain normoglycaemic on their natural diet.
(e) animals with gender selective differences in pancreatic islet or β-cell mass; or
(f) closely related animals, such as C57BI/6 and C57BI/Ks mice which show differences in pancreatic islet or β-cell mass.

8. The method of claim 1, wherein differential levels of islet cell or β-cell mass or function are induced by modifying the diet of pregnant animals or by comparing pregnant and non-pregnant animals in the paradigm.

9. The method of any one of the preceding claims, wherein the reduced levels of pancreatic islet or β-cell function are the result of non-insulin dependent diabetes (type 2 diabetes), syndrome X (insulin resistance syndrome) or gestational diabetes.

10. The method of any one of the preceding claims, wherein the increased levels of pancreatic islet or β-cell function in the subjects having increased levels of pancreatic islet or β-cell function are obtained by treatment with an insulin sensitiser drug, dietary restriction or exercise.

11. The method of claim 10, wherein the insulin sensitising drug is thiazolidinedione insulin sensitiser, a non-thiazolidinedione acting as an agonist or partial agonist of the PPAR gamma nuclear receptor, or a β ₃-adrenoceptor agonist or leptin.

12. The method of claim 11, wherein the thiazolidinedione insulin sensitiser is rosiglitazone (BRL 49653).

13. The method of any one of the preceding claims, wherein the subjects having an increased level of pancreatic islet or β-cell function are pregnant animals, or wherein the increased levels of pancreatic islet or β-cell function in the subjects having increased levels of pancreatic islet or β-cell function are obtained by administration of an insulin secretagogue peptide or drug.

14. The method of claim 13, wherein the insulin secretagogue is GLP-1 or a stable GLP-1 analogue or exendin 4.

15. The method of claim 13 or claim 14, wherein the insulin secretagogue further stimulates insulin production and/or the genesis of islet cells.

16. The method of any one of the preceding claims, wherein the paradigm is established using two-dimensional gel electrophoresis carried out on the relevant tissue or a protein containing extract thereof.

17. The method of any one of the preceding claims, further comprising:
(a) the step of isolating a differentially expressed protein identified in the method; and/or
(b) the step of characterising the isolated protein.

18. The method of any one of the preceding claims, wherein the differentially expressed protein or proteins comprise one or more of Tropomyosin isoform 1 (SWISS-PROT AC P46901), Fatty acid binding protein (adipocyte) (SWISS-PROT AC P04117), Profilin (SWISS-PROT AC P10924) and Carboxypeptidase B (SWISS-PROT AC P19223).

19. The method of claim 18, further comprising:
(a) using the protein in an assay for specific binding partners of the protein; and/or
(b) using the protein in an assay to screen for agonists or antagonists of the protein.

20. The method of any one of claims 1 to 19, wherein the agents or proteins are screened using a high throughput screening method.

21. A method of determining the nature or degree of pancreatic islet or β-cell dysfunction in a human or animal test subject, the method comprising:
(a) establishing a paradigm in which at least one protein is differentially expressed in relevant tissue from, or representative of, subjects having differential levels of pancreatic islet or β-cell function, the subjects comprising
(i) normal subjects who have and have not been treated with an agent which improves pancreatic islet or β-cell function, and
(ii) affected subjects having reduced or increased pancreatic islet or β-cell function relative to the normal subjects, wherein said affected subjects have and have not been treated with an agent which improves pancreatic islet or β-cell function,
wherein said differentially expressed protein is differentially expressed between said affected and normal subjects who have not been treated with the agent which improves pancreatic islet or β-cell function, and is differentially expressed between said affected subjects who have and have not been treated with the agent which improves pancreatic islet or β-cell function, but is not differentially expressed between said normal subjects who have and have not been treated with the agent which improves pancreatic islet or β-cell function;
(b) providing a sample of the tissue which has been obtained from the test subject;
(c) determining the presence, absence or degree of expression of the differentially expressed protein or proteins in the sample; and
(d) relating the determination to the nature or degree of the pancreatic islet or β-cell function by reference to a previous correlation between such a determination and clinical information.

22. The method of claim 21, wherein the sample is a tissue sample or body fluid sample or urine.

23. The method of claim 21 or claim 22, wherein in the paradigm at least four proteins are differentially expressed, providing a multi-protein fingerprint of the nature or degree of the pancreatic islet or β-cell dysfunction.

24. The method of any one of claims 21 to 23 which further comprises determining an effective therapy for treating the pancreatic islet or β-cell dysfunction.

25. The method of any one of claims 21 to 24, wherein the differentially expressed protein or proteins comprise one or more of Tropomyosin isoform 1 (SWISS-PROT AC P46901), Fatty acid binding protein (adipocyte) (SWISS-PROT AC P04117), Profilin (SWISS-PROT AC P10924) and Carboxypeptidase B (SWISS-PROT AC P19223).

26. A method according to any one of the preceding claims wherein the agent which improves pancreatic islet or β-cell function is an insulin sensitiser such as a thiazolidinedione, rosiglitazone, pioglitazone or troglitazone, an oxazolidadione JTT501, a non-thiazolidinedione PPAR gamma activator, an RXR activator that forms a heterodimer with PPAR gamma, metformin or a β₃-adrenoceptor agonist.

## Patentansprüche

1. Verfahren zum Screenen eines Prüfmittels zur Bestimmung seiner Nützlichkeit bei der Behandlung eines Leidens, das durch eine Dysfunktion der Langerhans-Inseln oder β-Zellen **gekennzeichnet** ist, wobei das Verfahren Folgendes umfasst:
(a) das Erstellen eines Paradigmas, worin zumindest ein Protein in relevantem Gewebe, das von Individuen mit unterschiedlichen Funktionswerten der Langerhans-Inseln oder β-Zellen stammt oder für solche repräsentativ ist, unterschiedlich exprimiert wird, wobei die Individuen folgende umfassen:
(i) normale Individuen, die mit einem Mittel, das die Funktion der Langerhans-Inseln oder β-Zellen verbessert, behandelt wurden oder nicht, und
(ii) betroffene Individuen, die eine verringerte oder gesteigerte Funktion der Langerhans-Inseln oder β-Zellen im Vergleich zu normalen Individuen aufweisen, worin die betroffenen Individuen mit dem Mittel, das die Funktion der Langerhans-Inseln oder β-Zellen verbessert, behandelt wurden oder nicht,
worin das unterschiedlich exprimierte Protein von den betroffenen und normalen Individuen, die nicht mit dem Mittel, das die Funktion der Langerhans-Inseln oder β-Zellen verbessert, behandelt wurden, unterschiedlich exprimiert wird und von den betroffenen Individuen, die mit dem Mittel, das die Funktion der Langerhans-Inseln oder β-Zellen verbessert, behandelt wurden oder nicht, unterschiedlich exprimiert wird, von den normalen Individuen, die mit dem Mittel, das die Funktion der Langerhans-Inseln oder β-Zellen verbessert, behandelt wurden oder nicht, aber nicht unterschiedlich exprimiert wird;
(b) das Bereitstellen einer Probe von relevantem Gewebe, das von einem Testindividuum mit verringerter Funktion der Langerhans-Inseln oder β-Zellen stammt oder für ein solches repräsentativ ist, wobei dieses Individuum oder das Gewebe mit dem gescreenten Prüfmittel behandelt wurde;
(c) das Bestimmen der Gegenwart, der Abwesenheit oder des Grads der Expression des unterschiedlich exprimierten Proteins oder der unterschiedlich exprimierten Proteine im Gewebe, das vom behandelten Testindividuum stammt oder für dieses repräsentativ ist; und
(d) das Auswählen oder Aussondern des Mittels je nach Ausmaß, in dem es die Expression, Aktivität oder Menge des unterschiedlich exprimierten Proteins oder der unterschiedlich exprimierten Proteine im behandelten Testindividuum mit verringerter Funktion der Langerhans-Inseln oder β-Zellen verändert.

2. Verfahren nach Anspruch 1, worin das Testmittel ausgewählt wird, wenn es die Expression des unterschiedlich exprimierten Proteins oder der unterschiedlich exprimierten Proteine in Richtung der Expression eines Individuums mit einer normaleren Funktion der Langerhans-Inseln oder β-Zellen abändert.

3. Verfahren nach Anspruch 1 oder Anspruch 2, worin das Prüfmittel ausgewählt wird, wenn es die Expression des Proteins oder der Proteine in die des normalen Individuums abändert.

4. Verfahren nach einem der Ansprüche 1 bis 3, worin die Dysfunktion der Langerhans-Inseln oder β-Zellen das Ergebnis einer Störung ist, die zur einer Verringerung der Masse der Langerhans-Inseln oder β-Zellen und/oder zu einer Verringerung der biologischen Aktivität der Langerhans-Inseln oder β-Zellen führt.

5. Verfahren nach einem der vorangegangenen Ansprüche, worin das Paradigma auf Gewebe von nicht insulinabhängigen diabetischen Individuen und normalen Individuen basiert.

6. Verfahren nach einem der vorangegangenen Ansprüche, worin das relevante Gewebe aus Langerhans-Inseln beseht.

7. Verfahren nach Anspruch 1, worin das Paradigma auf Folgendem basiert:
(a) Tieren, die zusammen mit schlanken Wurfgeschwistern Modelle für nicht insulinabhängigen Diabetes als Ergebnis einer genetischen Mutation, wie z.B. ob/ob, db/db, agouti, fat, fa/fa, darstellen; oder
(b) Tieren, bei denen die Dysfunktion der Inseln oder β-Zellen durch eine Diätbehandlung verschlimmert wird; oder
(c) dem Nachwuchs von trächtigen Tieren, die proteinarme Kost erhielten, worin gegebenenfalls die dem Nachwuchs nach der Entwöhnung verabreichte Kost außerdem eine fettreiche Kost ist;
(d) Wüstennagetieren, wie z.B. Stachelmäusen oder Sandratten, die bei normaler Laborkost Diabetes entwickeln, bei ihrer natürliche Kost jedoch normoglykämisch bleiben;
(e) Tieren mit geschlechtsspezifischen Unterschieden in der Masse der Langerhans-Inseln oder β-Zellen; oder
(f) eng verwandten Tieren, wie z.B. C57BI/6- und C57BI/Ks-Mäusen, die Unterschiede in der Masse der Langerhans-Inseln oder β-Zellen aufweisen.

8. Verfahren nach Anspruch 1, worin unterschiedliche Masse- oder Funktionswerte der Langerhans-Inseln oder β-Zellen durch Modifikation der Kost von trächtigen Tieren oder durch Vergleichen von trächtigen und nicht trächtigen Tieren im Paradigma induziert werden.

9. Verfahren nach einem der vorangegangenen Ansprüche, worin die verringerten Funktionswerte der Langerhans-Inseln oder β-Zellen das Ergebnis von nicht insulinabhängigem Diabetes (Typ-2-Diabetes), Syndrom X (insulinresistentes Syndrom) oder Trächtigkeitsdiabetes sind.

10. Verfahren nach einem der vorangegangenen Ansprüche, worin die erhöhten Funktionswerte der Langerhans-Inseln oder β-Zellen in den Individuen, die erhöhte Funktionswerte der Langerhans-Inseln oder β-Zellen aufweisen, durch Behandlung mit einem Insulinsensibilisierungsmittel, Kosteinschränkungen oder Bewegung erhalten werden.

11. Verfahren nach Anspruch 10, worin das Insulinsensibilisierungsmittel Thiazolidindion-Insulinsensibilisator, ein Nicht-Thiazolidindion, das als Agonist oder partieller Agonist des PPAR-Gamma-Kernrezeptors agiert, oder ein β₃-Adrenoceptor-Agonist oder Leptin ist.

12. Verfahren nach Anspruch 11, worin der Thiazolidindion-Insulinsensibilisator Rosiglitazon (BRL 49653) ist.

13. Verfahren nach einem der vorangegangenen Ansprüche, worin die Individuen mit erhöhten Funktionswerten der Langerhans-Inseln oder β-Zellen trächtige Tiere sind oder worin die erhöhten Funktionswerte der Langerhans-Inseln oder β-Zellen in den Individuen mit erhöhten Funktionswerten der Langerhans-Inseln oder β-Zellen durch Verabreichung eines Insulinsekretagogpeptids oder -arzneimittels erhalten werden.

14. Verfahren nach Anspruch 13, worin das Insulinsekretagog GLP-1 oder ein stabiles GLP-1-Analogon oder Exendin 4 ist.

15. Verfahren nach Anspruch 13 oder 14, worin das Insulinsekretagog weiters die Insulinproduktion und/oder die Bildung von Insel-Zellen stimuliert.

16. Verfahren nach einem der vorangegangenen Ansprüche, worin das Paradigma unter Verwendung von zweidimensionaler Gelelektrophorese erstellt wird, die am relevanten Gewebe oder an einem proteinhältigen Extrakt davon durchgeführt wird.

17. Verfahren nach einem der vorangegangenen Ansprüche, weiters Folgendes umfassend:
(a) den Schritt des Isolierens eines unterschiedlich exprimierten Proteins, das im Verfahren identifiziert wurde; und/oder
(b) den Schritt des Charakterisierens des isolierten Proteins.

18. Verfahren nach einem der vorangegangenen Ansprüche, worin das unterschiedlich exprimierte Protein oder die unterschiedlich exprimierten Proteine eines oder mehrere von Tropomyosin-Isoform-1 (SWISS-PROT AC P46901), einem fettsäurebindenden Protein (Adipozyt) (SWISS-PROT AC P04117), Profilin (SWISS-PROT AC P10924) und Carboxypeptidase B (SWISS-PROT AC P19223) umfasst.

19. Verfahren nach Anspruch 18, weiters Folgendes umfassend:
(a) die Verwendung des Proteins in einem Test für spezifische Bindungspartner des Proteins; und/oder
(b) die Verwendung des Proteins in einem Test zum Screenen auf Agonisten oder Antagonisten des Proteins.

20. Verfahren nach einem der Ansprüche 1 bis 19, worin die Wirkstoffe oder Proteine unter Verwendung eines Hochdurchsatz-Screeningverfahrens gescreent werden.

21. Verfahren zur Bestimmung der Art oder des Grads der Dysfunktion der Langerhans-Inseln oder β-Zellen in einem menschlichen oder tierischen Testindividuum, wobei das Verfahren Folgendes umfasst:
(a) das Erstellen eines Paradigmas, worin zumindest ein Protein in relevantem Gewebe, das von Individuen mit unterschiedlichen Funktionswerten der Langerhans-Inseln oder β-Zellen stammt oder für solche repräsentativ ist, unterschiedlich exprimiert wird, wobei die Individuen folgende umfassen:
(i) normale Individuen, die mit einem Mittel, das die Funktion der Langerhans-Inseln oder β-Zellen verbessert, behandelt wurden oder nicht, und
(ii) betroffene Individuen, die eine verringerte oder gesteigerte Funktion der Langerhans-Inseln oder β-Zellen im Vergleich zu normalen Individuen aufweisen, worin die betroffenen Individuen mit dem Mittel, das die Funktion der Langerhans-Insein oder β-Zellen verbessert, behandelt wurden oder nicht,
worin das unterschiedlich exprimierte Protein von den betroffenen und normalen Individuen, die nicht mit dem Mittel, das die Funktion der Langerhans-Inseln oder β-Zellen verbessert, behandelt wurden, unterschiedlich exprimiert wird und von den betroffenen Individuen, die mit dem Mittel, das die Funktion Langerhans-Inseln oder β-Zellen verbessert, behandelt wurden oder nicht, unterschiedlich exprimiert wird, von den normalen Individuen, die mit dem Mittel, das die Funktion der Langerhans-Inseln oder β-Zellen verbessert, behandelt wurden oder nicht, aber nicht unterschiedlich exprimiert wird;
(b) das Bereitstellen einer Probe von Gewebe, das von dem Testindividuum erhalten wurde;
(c) das Bestimmen der Gegenwart, der Abwesenheit oder des Grads der Expression des unterschiedlich exprimierten Proteins oder der unterschiedlich exprimierten Proteine in der Probe; und
(d) das In-Beziehung-Setzen der Bestimmung mit der Art oder dem Grad der Funktion der Langerhans-Inseln oder β-Zellen durch Verweis auf eine frühere Korrelation zwischen solch einer Bestimmung und klinischer Information.

22. Verfahren nach Anspruch 21, worin die Probe eine Gewebeprobe oder eine Körperflüssigkeitsprobe oder Urin ist.

23. Verfahren nach Anspruch 21 oder Anspruch 22, worin im Paradigma zumindest vier Proteine unterschiedlich exprimiert werden, was einen Multiprotein-Fingerprint der Art oder des Grads der Dysfunktion der Langerhans-Inseln oder β-Zellen bereitstellt.

24. Verfahren nach einem der Ansprüche 21 bis 23, das außerdem das Bestimmen einer effektiven Therapie zur Behandlung der Dysfunktion der Langerhans-Inseln oder β-Zellen umfasst.

25. Verfahren nach einem der Ansprüche 21 bis 24, worin das unterschiedlich exprimierte Protein oder die unterschiedlich exprimierten Proteine eines oder mehrere von Tropomyosin-Isoform-1 (SWISS-PROT AC P46901), einem fettsäurebindenden Protein (Adipozyt) (SWISS-PROT AC P04117), Profilin (SWISS-PROT AC P10924) und Carboxypeptidase B (SWISS-PROT AC P19223) umfasst.

26. Verfahren nach einem der vorangegangenen Ansprüche, worin das Mittel, das die Funktion der Langerhans-Inseln oder β-Zellen verbessert, ein Insulinsensibilisierungsmittel wie z.B. Thiazolidindion, Rosiglitazon, Pioglitazon oder Troglitazon, ein Oxazolidadion JTT501, ein Nicht-Thiazolidindion-PPAR-Gamma-Aktivator, ein RXR-Aktivator, der ein Heterodimer mit PPAR-Gamma bildet, Metformin oder ein β₃₋Adrenoceptor-Agonist ist.

## Revendications

1. Procédé de criblage d'un agent à l'essai pour déterminer son utilité dans le traitement d'un état **caractérisé par** un dysfonctionnement des îlots pancréatiques ou des cellules β, ledit procédé comprenant les étapes consistant à:
(a) établir un paradigme dans lequel au moins une protéine est exprimée de manière différentielle dans un tissu pertinent ou représentatif de sujets ayant des niveaux différentiels de fonction des îlots pancréatiques ou des cellules β, les sujets comprenant:
(i) des sujets normaux ayant et n'ayant pas été traités avec un agent qui améliore la fonction des îlots pancréatiques ou des cellules β, et
(ii) des sujets affectés ayant une fonction réduite ou accrue des îlots pancréatiques ou des cellules β relativement aux sujets normaux, lesdits sujets affectés ayant et n'ayant pas été traités avec l'agent qui améliore la fonction des îlots pancréatiques ou des cellules β,
ladite protéine exprimée de manière différentielle étant exprimée de manière différentielle entre lesdits sujets affectés et lesdits sujets normaux qui n'ont pas été traités avec l'agent qui améliore la fonction des îlots pancréatiques ou des cellules β et étant exprimée de manière différentielle entre lesdits sujets affectés qui ont et n'ont pas été traités avec l'agent qui améliore la fonction des îlots pancréatiques ou des cellules β, mais n'étant pas exprimée de manière différentielle entre lesdits sujets normaux qui ont et n'ont pas été traités avec l'agent qui améliore la fonction des îlots pancréatiques ou des cellules β;
(b) fournir un échantillon de tissu pertinent qui a été obtenu à partir d'un sujet à l'essai ayant une fonction réduite des îlots pancréatiques ou des cellules β, ou représentatif de ce sujet, qui a ou n'a pas été traité avec l'agent à l'essai qui est criblé;
(c) déterminer la présence, l'absence ou le degré d'expression de la protéine ou des protéines exprimées de manière différentielle dans le tissu provenant du sujet de test traité ou représentatif de ce sujet; et
(d) sélectionner ou rejeter l'agent selon l'ampleur suivant laquelle il modifie l'expression, l'activité ou la quantité de la ou des protéines exprimées de manière différentielle chez le sujet de test traité ayant une fonction réduite des îlots pancréatiques ou des cellules β.

2. Procédé selon la revendication 1, dans lequel l'agent à l'essai est sélectionné s'il convertit l'expression de la ou des protéines exprimées de manière différentielle pour la rapprocher de celle d'un sujet ayant une fonction plus normale de s îlots pancréatiques ou des cellules β.

3. Procédé selon la revendication 1 ou la revendication 2, dans lequel l'agent à l'essai est sélectionné s'il convertit l'expression de la ou des protéines en celle du sujet normal.

4. Procédé selon l'une quelconque des revendications 1 à 3, dans lequel le dysfonctionnement des îlots pancréatiques ou des cellules β résulte d'un trouble qui provoque une diminution de la masse des îlots pancréatiques ou des cellules β et/ou une diminution de l'activité biologique d'un îlot pancréatique ou des cellules β.

5. Procédé selon l'une quelconque des revendications précédentes, dans lequel le paradigme est fondé sur le tissu provenant de sujets diabétiques atteints de diabète non insulinodépendant et de sujets normaux.

6. Procédé selon l'une quelconque des revendications précédentes dans lequel le tissu pertinent est constitué d'îlots pancréatiques.

7. Procédé selon la revendication 1, dans lequel le paradigme est fondé sur:
(a) des animaux qui sont des modèles de diabète non insulinodépendant résultant d'une mutation génétique comme ob/ob, db/db, agouti, fat, fa/fa, avec des animaux similaires minces; ou
(b) des animaux chez lesquels le dysfonctionnement des îlots pancréatiques ou des cellules β est exacerbé par un traitement alimentaire; ou
(c) la descendance d'animaux gravides suivant un régime alimentaire pauvre en protéines, le régime alimentaire fourni à la descendance après sevrage étant facultativement un régime riche en graisses; ou
(d) des rongeurs du désert comme la souris épineuse et *Psammomys obesus* qui développent un diabète lorsqu'ils suivent un régime alimentaire normal de laboratoire mais restent normoglycémiques lorsqu'ils suivent leur régime alimentaire naturel.
(e) des animaux présentant des différences liées au sexe en matière de masse des îlots pancréatiques ou des cellules β; ou
(f) des animaux étroitement apparentés comme des souris C57BI/6 et C57BI/Ks qui présentent des différences de masse des îlots pancréatiques ou des cellules β.

8. Procédé selon la revendication 1, dans lequel des niveaux différentiels de cellules des îlots ou de masse ou de fonction des cellules β sont provoquées en modifiant le régime alimentaire d'animaux gravides ou en comparant des animaux gravides et non gravides dans le paradigme.

9. Procédé selon l'une quelconque des revendications précédentes, dans lequel les niveaux réduits de fonction des îlots pancréatiques ou des cellules β sont le résultat du diabète non insulinodépendant (diabète de type 2), du syndrome lié à l'X (syndrome de résistance à l'insuline) ou du diabète gestationnel.

10. Procédé selon l'une quelconque des revendications précédentes, dans lequel les niveaux accrus de fonction des îlots pancréatiques ou des cellules P chez les sujets ayant des niveaux ac crus de fonction des îlots pancréatiques ou des cellules P sont obtenus par traitement avec un potentialisateur des effets de l'insuline, une restriction alimentaire ou des exercices physiques.

11. Procédé selon la revendication 10, dans lequel le potentialisateur des effets de l'insuline est un potentialisateur des effets de l'insuline thiazolidinedione, un non thiazolidinedione agissant comme agoniste ou agoniste partiel du récepteur nucléaire PPAR gamma, ou un agoniste β3-adrénergique ou la leptine.

12. Procédé selon la revendication 11, dans lequel le un potentialisateur des effets de l'insuline thiazolidinedione est la rosiglitazone (BRL 49653).

13. Procédé selon l'une quelconque des revendications précédentes, dans lequel les sujets ayant un niveau accru de fonction des îlots pancréatiques ou des cellules P sont des animaux gravides, ou dans lequel les niveaux accrus de fonction des îlots pancréatiques ou des cellules β chez les sujets ayant des niveaux accrus de fonction des îlots pancréatiques ou des cellules P sont obtenus par administration d'un peptide ou d'un médicament sécrétagogues de l'insuline.

14. Procédé selon la revendication 13, dans lequel le sécrétagogue de l'insuline est le GLP-1 ou un analogue stable du GLP-1 ou l'exendine 4.

15. Procédé selon la revendication 13 ou la revendication 14, dans lequel le sécrétagogue de l'insuline stimule en outre la production d'insuline et/ou la genèse de cellules des îlots.

16. Procédé selon l'une quelconque des revendications précédentes, dans lequel le paradigme est établi en utilisant l'électrophorèse sur gel en deux dimensions réalisée sur le tissu pertinent ou un extrait de ce tissu contenant la protéine.

17. Procédé selon l'une quelconque des revendications précédentes, comprenant en outre:
(a) l'étape consistant à isoler une seconde protéine exprimée de manière différentielle identifiée dans le procédé; et/ou
(b) l'étape consistant à caractériser la protéine isolée.

18. Procédé selon l'une quelconque des revendications précédentes, dans lequel la protéine ou les protéines exprimées de manière différentielle comprennent une ou plusieurs de l'isoforme 1 de la tropomyosine (SWISS-PROT AC P46901), la protéine de liaison des acides gras (adipocyte) (SWISS-PROT AC P04117), la profiline (SWISS -PROT AC P10924) et la carboxypeptidase B (SWISS-PROT AC P19223).

19. Procédé selon la revendication 18, comprenant en outre les étapes consistant à:
(a) utiliser la protéine dans un test de détection de partenaires de liaison spécifiques de la protéine; et/ou
(b) utiliser la protéine dans un test pour cribler des agonistes ou des antagonistes de la protéine.

20. Procédé selon l'une quelconque des revendications 1 à 19, dans lequel les agents ou les protéines sont criblés en utilisant un procédé de criblage à haut débit.

21. Procédé pour déterminer la nature ou le degré du dysfonctionnement des îlots pancréatiques ou des cellules P chez un sujet de test humain ou animal, le procédé comprenant les étapes consistant à:
(a) établir un paradigme dans lequel au moins une protéine est exprimée de manière différentielle dans le tissu pertinent ou représentatif de sujets ayant des niveaux différentiels de fonction des îlots pancréatiques ou des cellules β, les sujets comprenant:
(i) des sujets normaux ayant et n'ayant pas été traités avec un agent qui améliore la fonction des îlots pancréatiques ou des cellules β, et
(ii) des sujets affectés ayant une fonction réduite ou accrue des îlots pancréatiques ou des cellules P relativement aux sujets normaux, lesdits suje ts affectés ayant et n'ayant pas été traités avec l'agent qui améliore la fonction des îlots pancréatiques ou des cellules β,
ladite protéine exprimée de manière différentielle étant exprimée de manière différentielle entre lesdits sujets affectés et lesdits sujets normaux qui n'ont pas été traités avec l'agent qui améliore la fonction des îlots pancréatiques ou des cellules P et étant exprimée de manière différentielle entre lesdits sujets affectés qui ont et n'ont pas été traités avec l'agent qui améliore la fonction des îlots pancréatiques ou des cellules β, mais n'étant pas exprimée de manière différentielle entre lesdits sujets normaux qui ont et n'ont pas été traités avec l'agent qui améliore la fonction des îlots pancréatiques ou des cellules β;
(b) fournir un échantillon de tissu qui a été obtenu à partir d'un sujet de test;
(c) déterminer la présence, l'absence ou le degré d'expression de la protéine ou des protéines exprimées de manière différentielle dans l'échantillon; et
(d) établir la relation entre cette détermination et la nature ou le degré de fonction des îlots pancréatiques ou des cellules β en référence à une corrélation antérieure entre cette détermination et des informations cliniques.

22. Procédé selon la revendication 21, dans lequel l'échantillon est un échantillon de tissu ou un échantillon de liquide organique ou de l'urine.

23. Procédé selon la revendication 21 ou la revendication 22, dans lequel, dans le paradigme, au moins quatre protéines sont exprimées de manière différentielle, fournissant une empreinte multi -protéiques de la nature ou du degré du dysfonctionnement des îlots pancréatiques ou des cellules β.

24. Procédé selon l'une quelconque des revendications 21 à 23 qui comprend en outre l'étape consistant à déterminer un traitement efficace pour traiter le dysfonctionnement des îlots pancréatiques ou des cellules β.

25. Procédé selon l'une quelconque des revendications 21 à 24, dans lequel la protéine ou les protéines exprimées de manière différentielle comprennent une ou plusieurs de l'isoforme 1 de la tropomyosine (SWISS-PROT AC P46901), la protéine de liaison des acides gras (adipocyte) (SWISS-PROT AC P04117), la profiline (SWISS -PROT AC P10924) et la carboxypeptidase B (SWISS-PROT AC P19223).

26. Procédé selon l'une quelconque des revendications précédentes dans lequel l'agent qui améliore la fonction des îlots pancréatiques ou des cellules β est un potentialisateur des effets de l'insuline comme un thiazolidinedione, la rosiglitazone, la pioglitazone ou la troglitazone, un oxazolidinedione JTT501, un activateur PPAR gamma non thiazolidinedione, un activateur RXR qui forme un hétérodimère avec PPAR gamma, la metformine ou un agoniste β3-adrénergique.
